Europäisches Patentamt

European Patent Office

Office européen des brevets

(11) Veröffentlichungsnummer: **0 270 496 B1**

(12) **EUROPÄISCHE PATENTSCHRIFT**

(45) Veröffentlichungstag der Patentschrift: **17.03.93**

(21) Anmeldenummer: **87810706.9**

(22) Anmeldetag: **30.11.87**

(51) Int. Cl.5: **C12N 15/07**, C12N 15/82, C12N 5/00, A01H 1/00

(54) **Verbessertes Verfahren zur Transformation von pflanzlichen Protoplasten.**

(30) Priorität: **05.12.86 CH 4866/86**

(43) Veröffentlichungstag der Anmeldung:
**08.06.88 Patentblatt 88/23**

(45) Bekanntmachung des Hinweises auf die
Patenterteilung:
**17.03.93 Patentblatt 93/11**

(84) Benannte Vertragsstaaten:
**AT BE CH DE ES FR GB GR IT LI LU NL SE**

(56) Entgegenhaltungen:
**EP-A- 0 164 575**

**BIOTECHNOLOGY, Band 3, Dezember 1985, Seiten 1099-1103; R.D. SHILLITO et al.: "High efficiency direct gene transfer to plants"**

**BIOTECHNOLOGY AND ECOLOGY OF POLLEN, INTERNATIONAL CONFERENCE, Amherst, Mass., 9.-11. Juli 1985, Seiten 65-70, Springer-Verlag, New York, US; I. NEGRUTIU et al.: "Attempts to transform for kanamycin-resistance in mature pollen of tobacco"**

**PLANT MOLECULAR BIOLOGY, Band 8, 1987, Seiten 363-373, Martinus Nijhoff Publishers,**

**Dordrecht, NL; I. NEGRUTIU et al.: "Hybrid genes in the analysis of transformation conditions"**

(73) Patentinhaber: **CIBA-GEIGY AG**
**Klybeckstrasse 141**
**CH-4002 Basel(CH)**

(72) Erfinder: **Negrutiu, Ioan, Dr.**
**4, Avenus des Mésanges**
**CH-1160 Bruxelles(CH)**
Erfinder: **Potrykus, Ingo, Dr.**
**Im Stigler 54**
**CH-4312 Magden(CH)**

**Beschreibung**

Die vorliegende Erfindung betrifft ein verbessertes Verfahren zur Transformation pflanzlicher Protoplasten mit einfachen rein chemischen Verfahrensmassnahmen.

Durch die Einführung neuer genetischer Information in pflanzliches Material können Pflanzen mit neuen und/oder verbesserten Eigenschaften erzeugt werden. Betrachtet man beispielsweise den raschen Anstieg der Weltbevölkerung und den damit verbundenen höheren Nahrungsmittel- und Rohstoffbedarf, so gehört die Steigerung des Ertrags von Nutzpflanzen sowie die vermehrte Gewinnung von pflanzlichen Inhaltsstoffen, insbesondere der Fortschritt auf dem Gebiet der Nahrungs- und Heilmittel, zu den dringlichsten Aufgaben der biologischen Forschung. In diesem Zusammenhang sind z.B. folgende wesentliche Aspekte zu nennen: eine Erhöhung der Resistenz von Nutzpflanzen gegen ungünstige Bodenverhältnisse oder Klimabedingungen, gegen Krankheiten und Schädlinge, eine gesteigerte Resistenz gegen Pflanzenschutzmittel wie Insektizide, Herbizide, Fungizide und Bakterizide, und eine günstige Veränderung des Nährstoffgehalts oder des Ernteertrags von Pflanzen. Solche wünschenswerten Effekte könnten allgemein durch Induktion oder vermehrte Bildung von Schutzstoffen, wertvollen Proteinen oder Toxinen herbeigeführt werden. Eine entsprechende Beeinflussung von pflanzlichem Erbgut kann beispielsweise dadurch erfolgen, dass man ein bestimmtes Fremdgen gezielt in Pflanzenzellen einschleust, ohne hierfür von konventionellen züchterischen Massnahmen Gebrauch zu machen.

Im Rahmen der vorliegenden Erfindung gelten folgende Definitionen:

| | |
|---|---|
| Pflanzliches Material: | in Kultur oder als solche lebensfähige pflanzliche Teile, wie Protoplasten, Zellen, Kallus, Gewebe, Embryonen, Pflanzenorgane, Knospen, Samen u.a. sowie ganze Pflanzen. |
| Gen: | Strukturgen mit flankierenden Expressionssignalen |
| Strukturgen: | Protein-kodierende DNA-Sequenz |
| Expressionssignale: | Promotorsignal und Terminationssignal |
| Pflanzenwirksames Expressionssignal: | Expressionssignal, das in Pflanzen funktionsfähig ist. |
| Promotorsignal: | die Transkription initiierendes Signal |
| Terminationssignal: | die Transkription terminierendes Signal |
| Enhancersignal: | die Transkription verstärkendes Signal |
| Replikationssignal: | die DNA-Replikation ermöglichendes Signal |
| Integrationssignal: | DNA-Sequenz, welche die Integration des Gens in die genomische DNA fördert |
| Hybridgen: | aus heterologen DNA-Sequenzen, d.h., DNA-Sequenzen verschiedenen Ursprungs aufgebautes Gen, wobei es sich sowohl um natürliche um c-DNA- als auch um synthetische DNA-Sequenzen handeln kann |
| Träger-DNA: | das Gen flankierende neutrale, d.h., an der Funktion des Gens nicht beteiligte DNA-Sequenz |
| Carrier DNA: | neutrale DNA ohne transformierendes Gen, welche dem transformierenden Gen beigemischt wird, um dieses gegen Nukleasen zu schützen. |
| Isoliertes Gen: | aus der originären DNA herausgetrennte DNA-Sequenz, welche ein einziges Protein kodiert |
| NPT-II-Gen: | Neomycin-3'-Phosphotransferase-Gen, Typ II von Transposon Tn 5 [[1] Rothstein, S.J. und W.S. Reznikoff, Cell 23, 191-199, (1981)] |
| Genomische DNA: | DNA eines Pflanzengenoms (total oder Teil davon) |
| c-DNA: | durch Reverse-Transkriptase hergestellte Kopie einer m-RNA |

Die Uebertragung von DNA-Sequenzen in Pflanzenzellen unter Verwendung von genetisch manipulierten Pflanzenbakterien ist aufgrund von Publikationen in der Fachliteratur, wie zum Beispiel [2] Nature, Vol. 303, 209-213 (1983); [3] Nature, Vol. 304, 184-187 (1983); [4] Scientific American 248(6), 50-59 (1983); [5] EMBO-Journal 2(6), 987-995 (1983); [6] Science 222, 476-482 (1983); [7] Science 223, 496-498 (1984); oder [8] Proc. Natl. Acad. Sci. USA 80, 4803-4807 (1983) bekannt. Bei den dort beschriebenen Verfahren werden die natürlichen pflanzeninfektiösen Eigenschaften dieser Bakterien ausgenutzt, um neues genetisches Material in Pflanzenzellen einzuschieusen. Zu diesem Zweck wurden bisher in erster Linie Pflanzenpathogene als Vektoren eingesetzt, wie beispielsweise Agrobacterium tumefaciens bzw. dessen Ti-Plasmid oder Cauliflower-Mosaik-Virus.

In jüngster Zeit entwickelte Verfahren ermöglichen jetzt auch die direkte Einschleusung eines Gens in pflanzliche Zellen ohne den Einsatz biologischer Vektoren. [9) Potrykus, I. et al., Plant Molec. Biol. Rep. 3, 117-128, (1985); 10) Shillito, R.D. et. al., Bio/Technology 3, 1099-1103, (1985)].

Diese, unter dem Schlagwort "Direkter Gentransfer" bekannt gewordenen Verfahren, erlauben eine Vektor-freie Transformation pflanzlicher Zellen ohne Verwendung von pflanzeninfektiösen Systemen, wie beispielsweise pflanzenpathogenen Bakterien, Viren, Insekten oder Pilzen.

Damit entfallen auch alle Einschränkungen, welche durch die Wirtsspezifität der Pathogene gegeben sind. Die Entwicklung der Pflanzen aus den transformierten Zellen wird durch die Anwendung der neuartigen Verfahren zur Transformation pflanzlicher Zellen nicht beeinträchtigt.

Ein Hauptproblem bei der Anwendung der Gentransformation liegt in der Schwierigkeit begründet, die transformierten Zellen oder Gewebe zu identifizieren.

Je geringer bei einer Gentransformation die Transformationshäufigkeit ist, desto schwieriger und aufwendiger ist das Auffinden der wenigen, aus den transformierten Zellen resultierenden Zellklone, unter der enormen Zahl nicht transformierter Klone. Die Anwendung üblicher Screening-Methoden ist daher bei geringer Transformationshäufigkeit nahezu oder gänzlich unmöglich, es sei denn, es handelt sich um ein Gen mit selektiver Marker-Funktion (z.B. Resistenz gegen eine bestimmte Substanz). Geringe Transformationshäufigkeit erfordert also bei Genen ohne selektionierbare Marker-Funktion einen enormen Aufwand.

Daher lassen sich bei Transformationen mit Genen ohne Markerfunktion die üblichen Screening-Methoden zur Selektionierung transformierter Zellklone erst dann sinnvoll und erfolgreich einsetzen, wenn die Transformationshäufigkeit in der Grössenordnung von $10^{-3}$ bis $10^{-2}$ liegt. Gegenwärtig lassen sich diese angestrebten hohen Transformationsraten nur bei Anwendung der Elektroporation, eventuell in Verbindung mit anderen in der mikrobiologischen Forschung zur Genübertragung angewendeten Verfahren, wie beispielsweise Poly-L-Ornithin- oder Poly-L-Lysin-Behandlung, Liposomenfusion, DNA-Proteinkomplexierung, Ladungsänderung an der Protoplastenmembran, Fusion mit mikrobiellen Protoplasten oder Calciumphosphat-Copräzipitation, und insbesondere Polyethylenglykolbehandlung und Hitzeschock-Methode (heat shock), erreichen [10) Shillito et al., Bio/Technology 3, 1099 - 1103 (1985)]. Dagegen konnten bisher, bei Anwendung rein chemischer Verfahrensmassnahmen, nur reproduzierbare Transformationsraten in einem Grössenordnungsbereich bis $10^{-5}$ erzielt werden.

Bei der Elektroporation [11) Neuman, E. et al., The EMBO Journal 7, 841-845 (1982), 10) Shillito et al., Bio/Technology 3, (1985)] werden Protoplasten in einer Mannit/Calcium bzw. einer Mannit/Magnesium-Lösung durch Entladen eines Kondensators über die Suspensionsflüssigkeit kurzzeitig mit einem Spannungs-Impuls von hoher Intensität beaufschlagt. Hierdurch wird eine Polarisierung der Protoplastenmembran und eine reversible Oeffnung von Poren in der Membran bewirkt, wodurch der Uebertritt der DNA in die Zelle erleichtert wird.

Dieses Verfahren besitzt jedoch zahlreiche Nachteile und ist bestimmten Beschränkungen unterworfen.

Zum einen ist für die Durchführung der Transformation mit Hilfe der Elektroporation ein relativ grosser apparativer Aufwand nötig, der mit einem entsprechenden Kosten- und Arbeitsanfall verbunden ist. Zum anderen ist die Anwendung dieses Verfahrens aufgrund der hohen Empfindlichkeit der pflanzlichen Protoplasten nur innerhalb eines sehr engen Grenzbereiches möglich. Um die Lebensfähigkeit der transformierten Zellen zu gewährleisten, können bestimmte Parameter wie beispielsweise die Spannungs-, Kapazitäts- und Feldstärkewerte, daher nur innerhalb dieser engen Grenzen variiert werden (z.B. Spannungsbereich zwischen 1400-1700 V) [10) Shillito, R.D. et al, Bio/Technology 3, (1985)].

Diese Nachteile können überraschenderweise in Rahmen des erfindungsgemässen Verfahrens mit einfachen rein chemischen Verfahrensmassnahmen überwunden werden.

Die im Rahmen der vorliegenden Erfindung durchgeführten Untersuchungen über die Prinzipien, die für die Aufnahme und Integration von DNA in Pflanzenzellen verantwortlich sind, haben deutlich werden lassen, dass der Transformationsprozess von einer Vielzahl sich gegenseitig beeinflussender Parameter abhängig ist. Aufbauend auf diesen Untersuchungen kann nunmehr die Transformationsfrequenz durch Variation und optimale Abstimmung der für die Transformation relevanten Parameter, im Vergleich zu herkömmlichen Verfahren, so deutlich gesteigert werden, dass die eingangs erwähnten angestrebten Transformationsraten von $10^{-3}$ bis $10^{-2}$ und mehr ohne weiteres erreicht werden.

Mit Hilfe dieses auf rein chemischen Massnahmen beruhenden erfindungsgemässen Verfahrens kann neben der Transformationsfrequenz nun überraschenderweise auch die Reproduzierbarkeit sowie die Ueberlebensrate der behandelten Protoplasten sehr stark erhöht werden.

Während bisher mit rein chemischen Verfahren maximal Transformationsfrequenzen im Bereich von $10^{-5}$ erzielt werden konnten, ist man jetzt, bei Anwendung des erfindungsgemässen Verfahrens, in der Lage, reproduzierbare Transformationsfrequenzen in einer Grössenordnung bis zu wenigen Prozent zu erreichen, vergleichbar denen der Elektroporation.

Im Gegensatz zur Elektroporation handelt es sich bei dem erfindungsgemäss verbesserten Verfahren um eine Methode, die ohne besonderen aparativen Aufwand und daher kostengünstig und weniger arbeitsintensiv durchgeführt werden kann. Ein weiterer Vorteil im Vergleich zu Methoden, die eine Elektroporation erfordern, besteht in der neuartigen Möglichkeit, nunmehr grosse Mengen von pflanzlichen Protoplasten gleichzeitig transformieren zu können.

Vergleichbare Beschränkungen, wie sie zuvor für das Elektroporationsverfahren im Zusammenhang mit der Ueberlebensrate der behandelten Zellen diskutiert wurden, sind bei Anwendung des erfindungsgemässen Verfahrens nicht gegeben.

Die für die erfindungsgemässe Transformation bestimmenden Parameter sind über einen weiten Bereich variabel und beeinträchtigen die Transformationseffizienz und die Lebensfähigkeit der behandelten Protoplasten innerhalb dieses Bereiches nicht.

Darüberhinaus erweist sich die gesamte Protoplastenpopulation nach der Transformation unter Verwendung des erfindungsgemässen Verfahrens als weit weniger heterogen als im Falle einer Transformation mittels Elektroporation, sowohl was die Lebens- wie die Teilungsfähigkeit der behandelten Protoplasten angeht. So lassen sich bei Anwendung des erfindungsgemässen Verfahrens beispielsweise ohne weiteres Ueberlebensraten der behandelten Protoplasten von 80 % und mehr erreichen, wobei gleichzeitig deren Teilungsfähigkeit und damit die Möglichkeit zur Bildung neuer Kolonien erhalten bleibt. Die vergleichbaren Werte bezüglich der Ueberlebensrate bei Anwendung der Elektroporation leigen dagegen bei nur ca. 10 %.

Es hat sich nun wider Erwarten gezeigt, dass von einer Vielzahl von Parametern, die einzeln oder untereinander kombiniert, die Transformationshäufigkeitbeim direkten Gentransfer in Protoplasten beeinflussen können, durch die richtige Auswahl und Kombination nur weniger dieser Parameter bzw. Massnahmen, mit geringem technischen und zeitlichen Aufwand eine optimale Transformationsrate erzielt werden kann. Dabei kristallisierten sich die folgenden Parameter als wesentlich heraus:

1) DNA-Probe : Form, Grösse, Konzentration, Zeitpunkt der Applikation
2) Carrier DNA : Form, Konzentration, Grösse
3) $Mg^{2+}$ : Konzentration, Zeitpunkt der Applikation
4) Plasmamembran modifizierendes Agens : Konzentration, Zeitpunkt der Applikation.

Unter den für das erfindungsgemäss Verfahren essentiellen Faktoren sind in erster Linie die $Mg^{2+}$-Konzentration sowie die Konzentration des die Plasmamembran modifizierenden Agens von entscheidender Bedeutung für die Steigerung der Transformationseffizienz. Dabei beobachtet man eine synergistische Interaktion zwischen diesen beiden genannten Faktoren, was in der Folge zu einer starken Erhöhung der Transformationsfrequenz führt.

Weiterhin von Bedeutung sind der Zeitpunkt und die Reihenfolge der Applikation der $Mg^{2+}$-Ionen, des modifizierenden Agens sowie der transformierenden DNA.

Diese entscheidende Verbesserung und Vereinfachung des Verfahrens zum direkten Gentransfer in pflanzliche Protoplasten und letztlich zur Erzeugung genetisch veränderter Pflanzen lässt sich durch die folgenden erfindungswesentlichen Teilschritte erreichen:

- Isolierung der Protoplasten aus pflanzlichem Material und gegebenenfalls Inkubation der isolierten Protoplasten in einem geeigneten Nährmedium,
- Resuspendieren der isolierten Protoplasten in einer standardisierten Salzlösung,
- Ueberführung der isolierten Protoplasten aus der Salzlösung in ein für die Transformation optimiertes Inkubationsmedium, das $Mg^{2+}$-Ionen enthält.
- Zugabe der einzuschleusenden DNA sowie eines die Plasmamembran modifizierenden Agens.
- Inkubation von Protoplasten und DNA in Gegenwart einer die Plasmamembran modifizierenden Substanz über einen Zeitraum, der für eine Penetration der DNA in die Protoplasten ausreicht,
- Abtrennung der behandelten Protoplasten aus der Inkubationslösung sowie gegebenenfalls deren Resuspendierung in einer wässrigen $CaCl_2$-Lösung
- Abtrennung der Protoplasten aus der $CaCl_2$-Lösung
- Inkubation in einem für die weitere Protoplastenentwicklung geeigneten Kulturmedium und
- sofern gewünscht, Regenerierung vollständiger transformierter Pflanzen.

Die vorliegende Erfindung betrifft somit im wesentlichen ein verbessertes Verfahren zur Transformation pflanzlicher Protoplasten und, falls gewünscht, die Erzeugung ganzer, genetisch modifizierter Pflanzen durch Regeneration aus besagten transformierten Protoplasten, das sich dadurch kennzeichnet, dass man

- pflanzliche Protoplasten aus beliebigen Pflanzengeweben isoliert und gegebenenfalls in einem der üblicherweise für die Kultivierung pflanzlicher Protoplasten verwendeten Nährmedien, kultiviert;
- besagte Protoplasten vor der eigentlichen Transformation 20 Minuten bis 6 Stunden in einem Vorinkubationsmedium, enthaltend Erdalkali und/oder Alkalikationen, vorzugsweise $Ca^{2+}$-, $K^+$ und/oder $Na^+$-Ionen sowie eine geeignete C-Quelle, bie einer Temperatur von 4° bis 10°C vorinkubiert;

4

- die Protoplasten anschliessend aus dem Vorinkubationsmedium abtrennt und in dem eigentlichen Transformationsmedium, enthaltend als essentiellen Bestandteil 0.1 bis 60 mM, vorzugsweise 10 bis 30 mM $Mg^{2+}$- Ionen, in An- oder Abwesenheit von $Ca^{2+}$- Ionen, resuspendiert;
- unmittelbar danach eine DNA-Probe, enthaltend ein oder mehrere Gene unter der Kontrolle von in Pflanzen aktiven Expressions-signalen sowie eine Träger-DNA, der Transformationslösung zufügt;
- einige Sekunden bis zu 20 Minuten, vorzugsweise 0,1 bis 10 Minuten, später ein die Plasmamembran modifizierendes Agenz hinzufügt;
- die Protoplasten und DNA-Probe in besagter Transformationslösung für einen Zeitraum inkubiert, der die Aufnahme der DNA in die Protoplasten gewährleistet; und
- sofern gewünscht, aus den transformierten Protoplasten ganze Pflanzen regeneriert.

Aus dieser Beschreibung geht klar hervor, dass die erfindungsgemässe Uebertragung der neuen Gene in die Pflanzenzellen auf direktem Wege ohne Benützung eines natürlichen pflanzeninfektiösen Systems, wie eines Pflanzenbakteriums, eines Pflanzenvirus oder Uebertragung durch Insekten oder phytopathogene Pilze erfolgt. Zu diesem Zweck werden die zu transformierenden Pflanzenprotoplasten direkt mit dem zu übertragenden Gen behandelt, indem man diese zunächst in eine geeignete Lösung einbringt, dort für eine gewisse Zeit vorinkubiert, anschliessend zusammen mit dem Fremdgen in das eigentliche Transformationsmedium überführt und dort für eine Zeitspanne, welche für die Aufnahme des Fremdgens in die Protoplasten ausreicht, belässt.

Als pflanzliche Protoplasten werden vorzugsweise jeweils solche einer einzigen Pflanzenart oder einer der Art untergeordneten systematischen Einheit verwendet.

Isolierte pflanzliche Protoplasten, welche sich auch als Ausgangsmaterial für isolierte Zellen und Gewebe eignen, können von beliebigen Teilen der Pflanze gewonnen werden, wie beispielsweise von Blättern, Keimlingen, Stengeln, Blüten, Wurzeln, Pollen oder Embryonen. Bevorzugt werden Blattprotoplasten verwendet. Die isolierten Protoplasten können auch aus Zellkulturen gewonnen werden. Methoden für die Isolierung von Protoplasten finden sich beispielsweise bei [12] Potrykus, I. and Shillito, R.D., Methods in Enzymology 118, 449 - 578 (1986).

Als Lösungen, in welchen die Protoplasten kultiviert werden, kommen vorzugsweise osmotisch stabilisierte Kulturmedien, wie sie üblicherweise für Protoplastenkulturen verwendet werden, in Betracht.

Es stehen bereits zahlreiche Kulturmedien zur Verfügung, die sich in einzelnen Medienkomponenten oder Gruppen solcher Komponenten unterscheiden. Alle Medien sind jedoch im allgemeinen nach dem folgenden Prinzip aufgebaut: sie enthalten eine Gruppe anorganischer Ionen im Konzentrationsbereich von ca. 10 mg/l bis zu einigen Hundert mg/l (sogenannte Makroelemente wie beispielsweise Nitrat, Phosphat, Sulfat, Kalium, Magnesium, Eisen), eine weitere Gruppe anorganischer Ionen in Mengen von maximal einigen mg/l (die sogenannten Mikroelemente wie beispielsweise Kobalt, Zink, Kupfer, Mangan), ferner eine Reihe von Vitaminen (beispielsweise Inosit, Folsäure, Thiamin), eine Energie- und Kohlenstoffquelle wie beispielsweise Saccharose oder Glukose, sowie Wachstumsregulatoren in Form natürlicher oder synthetischer Phytohormone aus den Klassen der Auxine und Cytokinine im Konzentrationsbereich von 0,01 bis 10 mg/l. Die Kulturmedien sind zudem osmotisch stabilisiert durch Zusatz von Zuckeralkoholen (beispielsweise Mannit) oder Zucker (beispielsweise Glukose) oder Salzionen (beispielsweise $CaCl_2$) und sind auf einen pH-Wert von 5,6 bis 6,5 eingestellt.

Eine nähere Beschreibung gängiger Kulturmedien findet sich beispielsweise in [13] Koblitz, H., Methodi-sche Aspekte der Zell- und Gewebezüchtung bei Gramineen unter besonderer Berücksichtigung der Getreide, Kulturpflanze XXII, 1974, 93-157.

Bevor die Protoplasten in das eigentliche Transformationsmedium überführt werden, erfolgt zunächst eine Vorinkubation in einem Medium, das die Protoplasten in optimaler Weise für die nachfolgende Transformation vorbereitet, was in einer deutlichen Erhöhung der erzielten Transformations- und Ueberlebensraten der behandelten Protoplasten zum Ausdruck kommt.

Unter gewissen Voraussetzungen ist es auch möglich, die Transformation direkt in besagtem Vorinkubationsmedium durchzuführen.

Es handelt sich bei besagtem Medium um eine standardisierte Salzlösung, die neben einer geeigneten C-Quelle, wie z.B. einem Zucker oder Zuckeralkohol wie Glucose oder Mannitol, verschiedene Salze, z.B. NaCl, $CaCl_2$, KCl, in einer Konzentration von 1 mM bis 200 mM erthält. Der pH-Wert dieser Salzlösung liegt vorteilhafterweise bei pH-Werten von pH 5 bis pH 8.

Kurz vor der beabsichtigten Transformation werden die Protoplasten aus der Vorinkubationslösung in das eigentliche Transformationsmedium überführt. Es handelt sich dabei um eine Mannitlösung, die $Mg^{2+}$-Ionen in einer Konzentration von 0.1 mM bis 60 mM, vorzugsweise in einer Konzentration von 10 mM bis 30 mM enthält. Der pH-Wert der Inkubationslösung liegt bei pH 5.6 bis pH 12, insbesondere bei pH 7 bis pH 10.

Unmittelbar nach Einbringen der isolierten Protoplasten in das Inkubationsmedium erfolgt die Zugabe der DNA-Probe in einer Konzentration von 2 $\mu$g/ml bis 20 $\mu$g/ml, vorzugsweise in einer Konzentration von 5 $\mu$g/ml bis 10 $\mu$g/ml.

Die DNA, bestehend aus einem Strukturgen und pflanzenwirksamen Expressionssignalen, wird vorteilhafterweise von neutralen DNA-Sequenzen (Träger-DNA) flankiert, welche die Integration des Gens in die genomische DNA der Pflanzenzelle ermöglichen. Es ist vorteilhaft, das Gen in linearisierter Form zu verwenden. Als besonders geeignet erwies sich im Verlauf der durchgeführten Experimente eine Träger-DNA Konzentration von 50 $\mu$g/ml bis 70 $\mu$g/ml bei einer durchschnittlichen Grösse zwischen 4 kb und 40 kb.

Es ist weiterhin vorteilhaft, neutrale DNA wie beispielsweise tierische oder pflanzliche DNA, Lambda DNA, Plasmid DNA oder jede beliebige andere DNA, die für die Durchführung des erfindungsgemässen Verfahrens geeignet ist, im Ueberschuss als Carrier-DNA einzusetzen, um das Gen gegen den Abbau durch Nukleasen zu schützen.

Einige Sekunden bis 20 Minuten, vorzugsweise 0.1 Minuten bis 10 Minuten nach der Zugabe der DNA in die Inkubationslösung erfolgt die Applikation von Polyethylenglykol bis zu einer Endkonzentration von 10 % bis 30 %. Hohe Transformationsraten erzielt man bei einer PEG-Konzentration von 20 % bis 28 %.

Neben Polyethylenglykol können aber auch andere höhere Alkohole oder alkoholartige Substanzen, die ebenfalls die Protoplastenmembran modifizieren und die beispielsweise auf dem Gebiet der Zellfusion zum Einsatz gelangen, in dem vorliegenden erfindungsgemässen Verfahren eingesetzt werden. Dabei handelt es sich beispielsweise um längerkettige, mehrwertige Alkohole, wie Polypropylenglykol (425 bis 4000 g/Mol), Polyvinylalkohol oder mehrwertige Alkohole, deren Hydroxygruppen teilweise oder vollständig veräthert sind, sowie pflanzenverträgliche, auf dem Agrargebiet gebräuchliche Detergentien, wie sie beispielsweise in folgenden Publikationen beschrieben werden:

[14] "Mc Cutcheon's Detergents and Emulsifiers Annual" MC Publishing Corp., Ridgewood New Jersey, 1981; Stache, H., "Tensid-Taschenbuch", Carl Hanser Verlag, München/Wien, 1981

Sofern Polyethylenglykol selbst eingesetzt wird (wie in den Beispielen 1 und 2), verwendet man vorzugsweise solches mit einem Molekulargewicht zwischen 1000 g/Mol und 10000 g/Mol, insbesondere zwischen 3000 g/Mol und 8000 g/Mol.

Von den vorgenannten Mitteln ist die Substanz Polyethylenglykol selbst bevorzugt, insbesondere eine Polyethylenglykollösung vom CMS-Typ, die einen relativ hohen Anteil an $Ca^{2+}$-Ionen besitzt. [[15] Negrutiu, I. et al., Theor. Appl. Genet. 72, 279 - 86, (1986a)].

Als besonders vorteilhaft für den Verlauf der Transformation erwies sich eine Inkubationsdauer mit Polyethylenglykol zwischen 20 Minuten und 6 Stunden.

Nach der in der oben angegebenen Weise durchgeführten Behandlung werden die Protoplasten in frischem Kulturmedium resuspendiert, wobei die Zelldichte vorteilhafterweise auf Werte zwischen $2 \times 10^4$ und $8 \times 10^4$ Protoplasten pro ml Kulturmedium eingestellt wird.

Bei der Verwendung von PEG in einer Konzentration von >20 % ist es zweckmässig, die Protoplasten nach erfolgter Transformation mit dem 2-10 fachen Volumen einer $Ca^{2+}$-Ionen enthaltenden Lösung schrittweise zu verdünnen und so durch anschliessende Sedimentation und Resuspension in frischem Kulturmedium vorhandene PEG-Reste auszuwaschen.

Als besonders vorteilhaft für die Transformationsergebnisse erwies sich dabei eine wässrige $CaCl_2$-Lösung, mit einer $Ca^{2+}$-Ionen-Konzentration von 0.1 M bis 1.0 M.

Das erfindungsgemässe Verfahren ist praktisch unbegrenzt anwendbar.

So können im Rahmen der vorliegende Erfindung beispielsweise chimäre genetische Konstruktionen verwendet werden, die als zentralen Bestandteil ein oder mehrere Strukturgene besitzen, die für neue nützliche und wünschenswerte Eigneschaften kodieren, und die in operabler Weise mit in pflanzlichen Zellen funktionellen Expressionssignalen verknüpft sind.

Für die Verwendung in dem erfindungsgemässen Verfahren sind in erster Linie alle die Gene geeignet, die in der pflanzlichen Zelle exprimiert werden und die der Pflanze eine nützliche und/oder gewünschte Geigenschaft verleihen, wie z.B. eine erhöhte Resistenz oder Toleranz gegenüber Pathogenen (z.B. phytophatogene Insekten, Pilze, Bakterien, Viren usw.) gegenüber Herbiziden, Insektiziden oder anderen Bioziden, gegenüber klimatischen Einflüssen und lokalen Besonderheiten (z.B. Hitze, Kälte, Wind, Trockenheit, Feuchtigkeit, besondere extreme Bodenverhälnise, osmotischer Stress usw.) oder mit einer erhöhten Bildung von Reserve- und Lagerstoffen in Blättern, Samen, Knollen, Wuzeln, Stengeln usw.

Ebenso werden von der vorliegenden Erfindung Gene umfasst, die für pharmazeutisch akzeptable aktive Wirksubstanzen, wie z.B. Alkaloide, Steroide, Hormone, Immunmodulatoren, und andere physiologisch aktive Substanzen kodieren.

So kann man beliebige Strukturgene pflanzlicher Herkunft, wie beispielsweise das Zein-Gen [16) Wienand, U., et al., Mol.Gen.Genet. 182, 440-444 (1981)], tierischer Herkunft, wie beispielsweise das TPA-Gen [Tissue-type plasminogen activator-Gen; 17) Pennica, D., et al., Nature 301, 214-221, (1983)], mikrobieller Herkunft wie beispielsweise das NPT-II-Gen, oder auch synthetischer Herkunft, wie beispielsweise das Insulin-Gen [18) Stepien, P., et al., Gene 24, 289-297 (1983)], in das Erbgut von Pflanzen übertragen, unter der Voraussetzung, dass die Strukturgene von Expressionssignalen flankiert werden, die in Pflanzen aktiv sind, wobei die Expressionssignale pflanzlicher, tierischer, mikrobieller oder synthetischer Herkunft sein können.

Unter Expressionssignale sind im Rahmen dieser Erfindung in erster Linie Promotor- und Terminationssequenzen zu verstehen, darüberhinaus aber auch weitere regulatorische Sequenzen der 5′- und der 3′-nichttranslatierten Regionen, die stromaufwärts bzw. stromabwärts der Strukturgen-Sequenzen gelegen sind.

Die Promotorsequenzen enthalten u.a. eine Erkennungsstelle für die RNA-Polymerase, an welche dieses Enzym spezifisch bindet und so die Transkription einleitet.

Verantwortlich für die Bindungsaffinität sind dabei bestimmte DNA-Sequenzen, die in prokaryontischen Promotoren gehäuft vorkommen. Diese sog. "Consensus"-Sequenzen findet man in der Regel in dem Sequenz-Bereich -10 bis -30 bezogen auf das ATG-Start-Kodon des Struktrugens. Es handelt sich dabei um zwei Hexanukleotid-Sequenzen, die als "Pribnov-Schaller-Box" bezeichnet werden und deren Nukleotidfolge innerhalb dieser Sequenzen sowie ihr Abstand zueinander die Affinität der DNA-Polymerase zum Promotor entscheidend beeinflussen.

In eukaryontischen Zellen kommt in diesem Zusammenhang der sog. "TATA" Box, einem Adenin- und Thyminreichen Sequenzabschnitt, der sich 20-30 Nukleotide stromaufwärts der Transkriptions-Initiationsstelle befindet, besondere Bedeutung zu.

Als Gene, die im Rahmen der vorliegenden Erfindung Verwendung finden können, kommen sowohl homologe als auch heterologe Gen(e) oder DNA sowie synthetische Gen(e) oder DNA gemäss der in Rahmen der vorliegenden Erfindung gemachten Definition in Betracht.

Die kodierende DNA-Sequenz kann ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA, wie auch die genomische DNA können aus verschiedenen Genen stammen. In jedem Fall können aber, sowohl die genomischen DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die mehr als einem Stamm, einer Varietät oder einer Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, müssen die kodierenden Gensequenzen zunächst in operabler Weise mit in pflanzlichen Zellen funktionsfähigen Expressions-Sequenzen verknüpft werden.

Die übertragenen Gene, bestehend aus Strukturgen und flankierenden Expressionssignalen, können dabei sowohl natürlich vorkommende Gene als auch Hybridgene sein. Bevorzugt werden im erfindungsgemässen Verfahren diejenigen Gene eingesetzt, deren Expressionssignale tierischer oder insbesondere pflanzlicher oder synthetischer Herkunft sind. Beispiele für derartige Gene sind:

a) vollständige Gene aus Pflanzen, bestehend aus dem Strukturgen mit seinen natürlichen Expressionssignalen;

b) vollsynthetische Gene, bestehend aus einem Strukturgen synthetischer Herkunft, flankiert von Expressionssignalen synthetischer Herkunft;

c) Strukturgene pflanzlicher Herkunft, flankiert von pflanzenwirksamen Expressionssignalen, wobei Strukturgen und Expressionssignale von verschiedenen Pflanzenarten stammen;

d) Strukturgene pflanzlicher Herkunft, flankiert von Expressionssignalen synthetischer Herkunft;

e) Strukturgene tierischer, mikrobieller oder synthetischer Herkunft, flankiert von Expressionssignalen pflanzlicher Herkunft; oder

f) Strukturgene tierischer oder mikrobieller Herkunft, flankiert von Expressionssignalen synthetischer Herkunft.

Die hybriden Genkonstruktionen im Rahmen der vorliegenden Erfindung enthalten somit neben dem (den) Strukturgen(en) Expressions-Signale, die sowohl Promoter- und Terminator-Sequenzen als auch weitere regulatorische Sequenzen der 3′ und 5′ nicht translatierte Regionen mit einschliessen.

Ganz besonders bevorzugt sind Strukturgene bakterieller Herkunft, flankiert von Expressionssignalen pflanzlicher Herkunft, insbesondere solchen mit Ursprung aus Pflanzenviren.

Dabei kann jeder Promotor und jeder Terminator, der in der Lage ist, eine Induktion der Expression einer kodierenden DNA-Sequenz (Strukturgen) zu bewirken, als Bestandteil der hybriden Gensequenz verwendet werden. Beispiele geeigneter Promotoren und Terminatoren sind z.B. solche der Nopalin-Synthase-Gene (nos), der Octopin-Synthase-Gene(ocs) sowie der Cauliflower Mosaik Virus Gene (CaMV).

Bevorzugt im Rahmen dieser Erfindung sind die 35S und 19S Expressions-Signale des CaMV Genoms, die mit Hilfe molekularbiologischer Methoden, wie sie z.B. bei [19] Maniatis et al., 1982 beschrieben sind, aus besagtem Genom isoliert und mit der kodierenden DNA-Sequenz verknüpft werden können.

Als Ausgangsmaterial für die 35S-Transkriptionskontroll-Sequenzen kann erfindungsgemäss z.B. das ScaI-Fragment des CaMV Stammes "S", das die Nukleotide 6808-7632 der Genkarte umfasst ([20] Frank G et al., 1980), verwendet werden.

Die 19S Promoter- und 5′ nicht-translatierte Region befindet sich auf einem Genomfragment zwischen der PstI Stelle (Position 5386) und der HindIII-Stelle (Position 5850) der CaMV Genkarte ([21] Hohn et al., 1982).

Die entsprechende Terminator- und 3' nichttranslatierte Region liegt auf einem EcoRV/BglII-Fragment zwischen Position 7342 und 7643 des CaMV Genoms.

Bei der 195 Promotor-Region handelt es sich um einen typisch eukaryontischen Promotor, der der kodierrenden Region des CaMV Gens VI vorgelagert ist und für die Expression des Gen VI-Produktes (Virus-Hüllenprotein) verantwortlich ist.

Ein weiterer wirksamer Vertreter eines in der pflanzlichen Zelle funktionellen Promotors, der Verwendung finden kann, ist ein überproduzierender Pflanzenpromotor. Diese Art von Promotoren sollte, sofern sie in operabler Weise mit der Gensequenz, welche für ein gewünschtes Genprodukt kodiert verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bis-phosphat-Carboxylase aus Sojabohnen [22] [Berry-Lowe et al., J. Mol. and Appl. Genet., 1: 483-498 (1982)] sowie den Promotor des Chlorophyll-a/b-Bindungsproteins ein. Diese beiden Promotoren sind dafür bekannt, dass sie in eukaryontischen Pflanzenzellen durch Licht induziert werden [siehe z.B. [23] Genetic Engineering of Plants, an Agricultural Perspective, A. Cashmore, Plenum, New York 1983, Seite 29-38; [24] Coruzzi G. et al., The Journal of Biological Chemistry, 258: 1399 (1983) und [25] Dunsmuir, P. et al., Journal of Molecular and Applied Genetics, 2: 285 (1983)].

Für eine Anwendung gemäss vorliegender Erfindung haben sich die Expressionssignale des Gens VI des Cauliflower-Mosaik-Virus als besonders vorteilhaft erwiesen.

Besonders bevorzugt im Rahmen dieser Erfindung sind die Expressionssignale des CaMV-Stammes CM 1841, dessen komplette DNA-Sequenz bei [26] Gardner et al, 1981 beschrieben ist.

Die Hybridgene werden nach an sich bekannten mikrobiologischen Verfahren hergestellt, wobei das Leseraster der Kodierung für die von der Pflanzenzelle zu produzierenden Proteine beibehalten wird. Derartige Methoden sind bekannt und werden beispielsweise in folgenden Publikationen beschrieben: [27] "Molecular Cloning", Maniatis, T., Fritsch, E.F. und J. Sambrook, Cold Spring Harbor Laboratory, 1982, und [20] "Recombinant DNA Techniques", Rodriquez, R.L. und R.C. Tait, Addison-Wesley Publishing Comp., London, Amsterdam, Don Mills. Ontario, Sydney, Tokyo, 1983.

Für die Integration des Fremdgens in die genomische DNA der Pflanzenzelle ist es vorteilhaft, wenn das Gen, bestehend aus Strukturgen und pflanzenwirksamen Expressionssignalen, von neutralen DNA-Sequenzen (Träger-DNA flankiert wird. Die Trager-DNA kann dabei aus zwei linearen DNA-Strängen bestehen, so dass die in die Pflanzenzelle einzuschleusende Konstruktion ein lineares DNA-Molekül ist. Die zur Genübertragung hergestellte DNA-Sequenz kann aber auch eine ringförmige Struktur (Plasmidstruktur) haben. Solche Plasmide bestehen aus einem DNA-Strang, in den das Fremdgen mit den Expressionsignalen integriert ist. Die Träger-DNA kann synthetischen Ursprungs sein oder durch Behandlung mit geeigneten Restriktionsenzymen aus natürlich vorkommenden DNA-Sequenzen gewonnen werden. So sind beispielsweise als Träger-DNA natürlich vorkommende Plasmide geeignet, die mit einem selektiven Restriktionsenzym geöffnet worden sind.

Ein Beispiel für ein derartiges Plasmid ist das frei erhältliche Plasmid pUC8 (beschrieben in [28] Messing, J. und J. Vieira, Gene 19, 269-276, 1982). Weiter sind als Träger-DNA auch Bruchstücke natürlich vorkommender Plasmide verwendbar. Beispielsweise ist als Träger-DNA die Deletionsmutante für Gen VI des Cauliflower-Mosaik-Virus einsetzbar.

Die Wahrscheinlichkeit der genetischen Transformation einer Pflanzenzelle kann durch verschiedene Faktoren erhöht werden. So steigt, wie man aus Versuchen mit Hefe weiss, die Anzahl der erfolgreichen stabilen Gentransformationen

1) mit steigender Anzahl von Kopien der neuen Gene pro Zelle,

2) bei Kombination eines Replikationssignals mit dem neuen Gen, sowie

3) bei Kombination von Integrationssequenzen mit dem neuen Gen.

Somit ist das erfindungsgemässe Verfahren besonders vorteilhaft anzuwenden, wenn das übertragene Gen mit in Pflanzenzellen wirksamen Replikations- und/oder Integrationssequenzen kombiniert ist.

Die Expression eines Gens in einer Pflanzenzelle ist abhängig von der Transkriptionshäufigkeit des Gens in eine Messenger-RNS-Sequenz. Es ist daher ebenfalls von Vorteil, wenn das neue Gen mit einem diese Transkription verstärkenden Enhancersignal kombiniert ist. Insbesondere sind diejenigen Verfahren hervorzuheben, bei denen ein Gen übertragen wird, das mit in Pflanzenzellen wirksamen Replikations-, Integrations- und Enhancersignalen kombiniert ist.

Weiterhin ist es von grossem verfahrenstechnischem Vorteil, wenn das übertragene Gen eine selektive Markerfunktion besitzt, d.h., dass die transformierten Pflanzenzellen von den nicht-transformierten Pflanzenzellen durch eine gezielte Selektionierung getrennt werden können. Eine derartige Markerfunktion erlaubt eine rationelle Arbeitsweise dadurch, dass nur diejenigen Pflanzenzellen durch übliche mikrobiologische Massnahmen zu Kalli oder vollständigen Pflanzen regeneriert werden müssen, in deren Erbgut ein zur Expression gelangendes Gen vorhanden ist, welches Marker-spezifische Selektionierungsmassnahmen gestattet.

Während als Pflanzenzellen, die als Ausgangsmaterialien für eine Transformation in Betracht zu ziehen sind, Protoplasten, Zellkulturzellen, Zellen in Pflanzengeweben, Pollen, Pollenschläuche, Eizellen, Embryosäcke oder Zygoten und Embryonen in unterschiedlichen Entwicklungsstadien zu nennen sind, sind Protoplasten wegen der direkten Einsatzmöglichkeit ohne weitere Vorbehandlungen bevorzugt.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae oder Gramineae und der Ordnung Leguminosae und hier vor allem der Familie Papilionaceae. Bevorzugt sind Vertreter der Familien Solanaceae, Cruciferae und Gramineae.

Besonders erwähnenswert sind Pflanzen der Gattungen Nicotiana, Petunia, Hyoscyamus, Brassica und Lolium, wie beispielsweise Nicotiana tabacum, Nicotiana plumbagenifolia, Petunia hybrida, Hyoscyamus muticus, Brassica napus, Brassica rapa und Lolium multiflorum.

Die Kulturpflanzen mit grossen Ernteerträgen wie Mais, Reis, Weizen, Gerste, Roggen, Hafer oder Hirse sind in erster Linie Objekt der Anstrengungen auf dem Gebiet der Transformation von Pflanzenzellen.

Alle Pflanzen, die durch Regeneration aus Protoplasten erzeugt werden können, lassen sich auch unter Anwendung des erfindungsgemässen Verfahrens transformieren. Vertreter der Familie Gramineae (Gräser), welche auch Getreide umfasst, konnten bisher nicht genetisch manipuliert werden. Es wurde nunmehr nachgewiesen, dass mit der vorstehend beschriebenen Methode der direkten Gentransformation auch Gramineenprotoplasten, also auch Getreidezellen, genetisch transformiert werden können. In gleicher Weise ist eine Transformation der Kulturpflanzen der Gattungen Solanum, Nicotiana, Brassica, Beta,Pisum,Phaseolus, Glycine, Helianthus, Allium, Avena, Hordeum, Oryzae, Setaria, Secale, Sorghum, Triticum, Zea, Musa, Cocos, Cydonia, Pyrus, Malus, Phoenix, Elaeis, Rubus, Fragaria, Prunus, Arachis, Secale, Panicum, Saccharum, Coffea, Camellia, Musa, Ananas, Vitis oder Citrus möglich und erwünscht, wenn auch die Gesamterträge und -anbauflächen hierfür weltweit geringer sind.

Die Regeneration von in Kultur gehaltenen Protoplasten zu ganzen Pflanzen ist bei [29] Evans, eta al., "Protoplast Isolation and Culture", in Handbook of Plant Cell Culture, 1: 124-176 (MacMillan Publishing Co. New York 1983); [30] MR Davey, "Recent Developments in the Culture and Regeneration of Plant Protoplasts", Protoplasts, 1983 - Lecture Proceedings, Seite 19-29, (Birkhäuser, Basel 1983); [31] PJ Dale, "Protoplast Culture and Plant Regenerations of Cereals and Other Recalcitrant Crops", in Protoplasts 1983 - Lecture Proceedings, Seite 31-41, (Birkhäuser, Basel 1983); und [32] H Binding, "Regeneration of Plants", in Plant Protoplasts, Seite 21-37, (CRC Press, Boca Raton 1985) und bei [12] Potrykus I and Shillito RD, Methods in Enzymology, Vol. 118, Plant Molecular Biology, eds. A. and H. Weissbach, Academic Press, Orlando, 1986, beschrieben.

Die Regenerationsverfahren unterscheiden sich von Pflanzenspezies zu Pflanzenspezies. Im allgemeinen aber wird zunächst eine Suspension von transformierten Protoplasten, Zellen oder von Gewebe, die zahlreiche Kopien der eingeschleusten Gene enthalten, hergestellt. Ausgehend von diesen Suspensionen kann anschliessend die Induktion der Embryobildung erfolgen. Man lässt die Entwicklung der Embryonen bis zum Stadium der Reife und Keimung fortschreiten, wie dies auch bei natürlicherweise vorkommenden Embryonen der Fall ist. In der Regel werden aber die Protoplasten in einem der bekannten Kulturmedien zur Teilung und Zellwandbildung angeregt. Es entstehen schliesslich Kalluskulturen, welche durch Behandlung mit bestimmten Wirkstoffen, wie z.B. Auxinen und Cytokininen zur Wurzel- bzw. Sprossbildung induziert werden können. Neben diesen Wuchsstoffen enthalten die Kulturmedien in der Regel verschiedene Aminosäuren. Es erweist sich dabei als vorteilhaft, dem Medium Glutaminsäure und Prolin zuzugeben, insbesondere bei Spezies wie Mais und Alfalfa. Die Spross- und Wurzelbildung erfolgt im allgemeinen simultan.

Die auf diese Weise gewonnenen Pflänzchen können anschliessend in Erde übertragen und in gleicher Weise wie normale Sämlinge weiterkultiviert werden.

Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wiederholbar.

Aufgrund neuer Enwicklungen auf dem Gebiet der in vitro Kultivierung von Pflanzen, vornehmlich im Bereich der Pflanzenregeneration, ist es inzwischen möglich, auch bei Vertretern aus der Familie der Gramineae, ausgehend von pflanzlichen Protoplasten, ganze Pflanzen zu regenerieren. Beispiele von erfolgreich durchgeführten Regenerationsexperimenten bei Gramineen sind u.a. bei [33)] Abdullah, R et al., Bio/Technology, 4: 1087-1090, 1986, [34)] Fujimura, T., et al., Plant Tissue Culture Lett, 2:74-75, 1985, [35)] Torijama, K., et al., Theor Appl Genet, 73:16-19, 1986, [36)] Yamada, Y., et al., Plant Cell Rep, 5:85-88, 1986 (Reis) sowie in der hängigen US-Patentanmeldung mit der Seriennummer 056,506, die am 24. Juni 1987 unter dem Titel "Regeneration of Zea mays plants from protoplast" eingereicht wurde, beschrieben.

Es können im Rahmen der vorliegenden Erfindung daher auch die folgenden Pflanzen verwendet werden: Lolium, Zea, Triticum, Sorghum, Saccharum, Bromus, Oryzae, Avena, Hordeum, Secale und Setaria.

Der Nachweis transformierter Gene kann auf an sich bekannte Weise erfolgen, beispielsweise durch Kreuzungsanalysen und molekularbiologische Untersuchungen, zu denen besonders die "Southern blot"-Analyse und Enzymaktivitätstests gehören.

Im Rahmen der Kreuzungsanalyse werden die reifen Pflanzen, die aus transformierten Pflanzenzellen herangezogen wurden, zunächst zur Samenproduktion mit sich selbst gekreuzt. Einige der Samen enthalten die eingeschleusten Gene, die für eine neue und wünschenswerte Eigenschaft kodieren, in einem Verhältnis, das genau den etablierten Gesetzen der Vererbung gehorcht. Diese Samen können zur Produktion von Pflanzen mit neuen und gewünschten Eigenschaften verwendet werden.

Homozygote Linien können durch wiederholte Selbstfertilisation und Herstellung von Inzuchtlinien gewonnen werden. Diese Inzuchtlinien können dann wiederum zur Entwicklung von Hybriden verwendet werden. Bei diesem Verfahren wird eine Inzuchtlinie mit einer anderen Inzuchtlinie gekreuzt.

Die "Southern blot"-Analyse kann beispielsweise folgendermassen durchgeführt werden: Die den transformierten Zellen oder Protoplasten entnommene DNA wird nach Behandlung mit Restriktionsenzymen einer Elektrophorese in 1 %-igem Agarose-Gel unterworfen, auf eine Nitrozellulosemembran [[37)] Southern, E.M., J.Mol.Biol. 98, 503-517 (1975)] transferiert und mit der nachzuweisenden DNA, welche einer Nick-Translation [[38)] Rigby, W.J., Dieckmann, M., Rhodes, C. und P. Berg, J.Mol. Biol. 113, 237-251, (1977)] unterworfen wurde (DNA-spezifische Aktivität von 5 x $10^8$ bis 10 x $10^8$ c.p.m./$\mu$g) hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNA wird durch Schwärzung eines Röntgenfilms während 24 bis 48 Stunden sichtbar gemacht.

Eine Untersuchung auf Enzymaktivität lässt sich - näher erläutert am Test auf Aminoglykosidphosphotransferase (Enzym für Kanamycinspezifische Phosphorylierung) - beispielsweise folgendermassen durchführen: Kallus- oder Blattstücke (100 bis 200 mg) werden in 20 $\mu$l Extraktionspuffer in einem Eppendorf-Zentrifugenröhrchen homogenisiert. Der Puffer ist eine Modifikation des von [5)] Herrera-Estrella, L., DeBlock, M., Messens, E., Hernalsteens, J.-P., Van Montagu, M. und J. Schell, EMBO J. 2, 987-995 (1983) verwendeten Puffers, in welchem Albumin aus Rinderblut weggelassen und 0,1 M Sucrose hinzugefügt worden sind. Die Extrakte werden 5 Minuten lang bei 12'000 g zentrifugiert und die überstehende Phase mit Bromphenolblau bis zu einer Endkonzentration von 0.004 % versetzt. Durch Elektrophorese in einem 10 %-igen, nicht denaturierenden Polyacrylamid-Gel werden die Proteine aus 35 $\mu$l der überstehenden Phase separiert. Das Gel wird mit einem Kanamycin und $\gamma$-$^{32}$P-markierte ATP enthaltenden Agarose-Gel überschichtet, inkubiert, und die phosphorylierten Reaktionsprodukte werden auf Whatman-p81-Phosphozellulose-Papier übertragen. Das Papier wird sechsmal mit deionisiertem Wasser von 90°C gewaschen und dann autoradiografiert.

EP 0 270 496 B1

Die nachfolgenden Beispiele dienen der näheren Illustration der vorliegenden Erfindung, ohne diese jedoch einzuschränken. Sie beschreiben die Konstruktion eines Hybridgens und dessen Einbau in Träger-DNA-Sequenzen mit cyclischem Charakter, die Uebertragung dieses Hybridgens in Pflanzenzellen, die Selektionierung der transformierten Pflanzenzellen und die Regeneration von vollständigen Pflanzen aus den transformierten Pflanzenzellen sowie deren kreuzungsgenetische und molekularbiologische Analyse.

In den nachfolgend aufgeführten Ausführungsbeispielen werden die folgenden Abkürzungen verwendet:

Medien:

| | |
|---|---|
| HeNa/F | [10 mM Hepes, pH 7.1, 5 mM $CaCl_2$, 150 mM NaCl, 0.2 M Mannit; [39] Fromm, M. et al., (1985)] |
| $K_3$ | [0.1 mg/l 2.4 D, 1,0 mg/l NAA, 0.2 mg/l BAP; [40] Shillito, R.D. et al. (1981); [41] Nagy, J.I. and Maliga, P., (1976)] |
| $KA_1 AO$ | [[42] Installe, P. et al., (1985)] |
| LS | [[43] Linsmaier, E.M., Skook, F., Physiologia Plantarum 18, 100-127, (1965)] |
| MaCa | [0.4 M Mannit, 15 mM $CaCl_2 x 2H_2O$, pH 5.6] |
| MaMg | [0.4 - 0.5 M Mannit, 15 mM $MgCl_2$, 0.1 % MES, pH 5.6] |
| M | [[42] Installe, P. et al., (1985)] |
| $MAP_1 AO$ | [[42] Installe, P. et al., (1985)] |
| MDs | [[44] Negrutiu, I. et al., (1983)] |
| RP | [[42] Installe, P. et al., (1985)] |
| R'SA | [[42] Installe, P. et al., (1985)] |
| T | [[45] Nitsch, J.P. et C. Nitsch, (1969)] |
| $W_5$ | [154 mM NaCl, 125 mM $CaCl_2 x 2H_2O$, 5 mM KCl, 5 mM Glucose, pH 5.6 - 6.0; [46] Menczel et al., (1981)] |

Chemikalien:

| | |
|---|---|
| BAP | Benzylaminopurin |
| 2.4 D | (2.4-Dichlorophenoxy)essigsäure |
| NAA | Naphthylessigsäure |
| EDTA | Ethylendiamin-N,N,N′,N′-tetraessigsäure |
| PEG CMS | Polyethylenglykol vom CMS-Typ [[15] Negrutiu, I. et al. (1986a)] |
| PEG 6R | Polyethylenglykol vom R - Typ [[10] Shillito, R.D. et al., (1985)] |
| Tris-HCl | $\alpha,\alpha,\alpha$-Tris-(hydroxymethyl)-methylamin-Hydrochlorid |
| NPT-II-Gen | Neomycin-3′-Phosphotransferase-Gen, Typ II |

Tabellen:

| | |
|---|---|
| ATF | Absolute Transformationsfrequenz |
| GPPL | Gesamt-Protoplastenzahl |
| RTF | Relative Transformationsfrequenz |
| UE | Ueberlebende Protoplasten nach erfolgter Transformationsbehandlung |
| ZT | Zahl der Transformanten |

Bei den in den nachfolgenden Ausführungsbeispielen gemachten Prozentangaben handelt es sich um Gewicht/Volumen-Angaben (w/v; volumetrisches Gewicht).

Kurzbeschreibung der Abbildungen

Abbildung 1 zeigt die Konstruktion der das NPT-II Strukturgen enthaltenden Plasmidvektoren pABD I und pABD II.

Abbildung 2 demonstriert den Einfluss der $MgCl_2$-Konzentration (A) sowie der $MgCl_2$/PEG-Interaktion (B) auf die Transformationsraten bei Nicotiana tabacum c.v. Petit Havanna $SR_1$ (A u. B) sowie bei Nicotiana plumbaginifolia (A).

11

Abb. A

Kurve a:   N. tabacum SR$_1$
Kurve b:   N. plumbaginifolia

Zu beachten ist die semilogarithmische Skaleneinteilung der MgCl$_2$-Konzentration (Abszisse). Die PEG-Konzentration liegt in diesem Fall bei 20 % (w/v).

Abb. B

Kurve a:   15 mM MgCl$_2$
Kurve b:   30 mM MgCl$_2$

Beispiel 1: Konstruktion des Plasmids pABDI

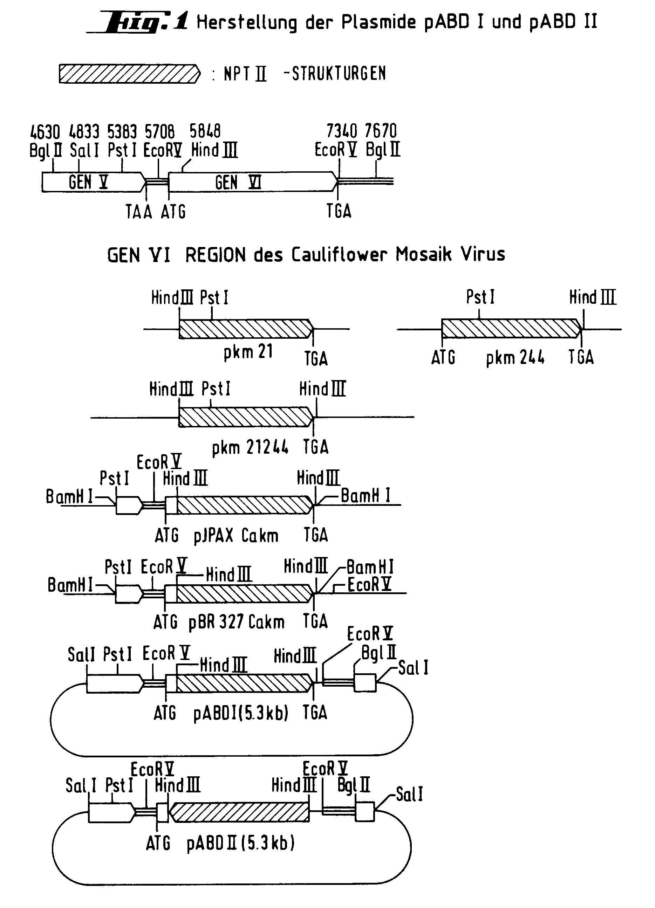

Man zerschneidet die frei zugänglichen Plasmide pkm 21 und pkm 244 [[47] Beck, E., et al., Gene 19, 327-336 (1982)] mit der Restriktions-Endonuclease PstI. Die Fragmente der Plasmide, welche für die Rekombination verwendet werden, werden durch Elektrophorese in 0.8%igem Agarose-Gel gereinigt. Das aus der Verbindung der Bruchstücke erhaltene Plasmid pkm 21244 enthält eine Kombination der 5'- und 3'-Bal 31 - Deletionen des NPT-II-Gens, wie von [47] Beck et al. in Gene 19, 327-336 (1982) beschrieben. Um das Promotersignal des Cauliflower-Mosaik-Virus mit dem HindIII-Fragment des Plasmids pkm 21244 zu verbinden, wird das Kupplungsplasmid pJPAX konstruiert. Das Kupplungsplasmid pJPAX wird aus den Plasmiden pUC8 und pUC9 [[28] Messing, J. and J. Vieira, Gene 19, 269-276 (1982)] erhalten. 10 Basenpaare von der Kupplungssequenz des Plasmids pUC9 werden durch Zerschneiden bei der HindIII- und der SalI-Position und nachfolgendem Auffüllen der haftfähigen Enden mit dem Polymerase-I-Klenow-Fragment [[48] Jacobsen, H., et al., Eur. J. Biochem. 45, 623, (1974)] und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SmaI, BamHI, SalI, PstI, HindIII, BamHI, XhoI und EcoRI. Die CaMV Gen VI-Promotor-Region, die mit dem NPT II Struktur-Gen verknüpft wird, stammt aus dem Genom des CaMV-Stammes CM4 184, einer Variante des CaMV Stammes CM 1841, dessen vollständige Nukleotid-Sequenz bei [26] Gardner RC et al., 1981 beschrieben ist. Die CaMV Promotor-Region wird in dem 6kb umfassenden Cosmid pHC79, einem Abkömmling des E. coli Plasmids pBR322 ([49] Hohns B. and Collins J., 1980) kloniert, wobei das CaMV Genom sowie das Cosid pHC79 mit Bst II geschnitten und die resultierenden Bruchstücke miteinander verknüpft werden. Die Verbindung des 5'-Expressionssignalsdes Cauliflower-Mosaik-Virus-Gens VI und des HindIII-Fragments des NPT-II-Gens wird beim Plasmid pJPAX durch Einfügung der Promoterregion des Cauliflower-Mosaik-Virus-Gens VI zwischen die PstI- und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm$^+$ und pJPAX Cakm$^-$ erhält. Um eine EcoRV-Sequenz in der Nähe des 3'Terminationssignals des NPT-II-Hybridgens zu schaffen, wird ein BamHI-Fragment des Plasmids pJPAX Cakm$^+$ in die BamHI-Position des Plasmids pBR 327 [[50] Soberon, X. et al., Gene 9, 287-305 (1980)] eingebaut. Man erhält das Plasmid pBR 327 Cakm. Das EcoRV-Fragment des Plasmids pBR 327 Cakm, das die neue DNA-Konstruktion enthält, wird verwendet, um die EcoRV-Region des Cauliflower-Mosaik-Virus-Gens VI zu ersetzen, welches an der SalI-Position im Plasmid pUC8 kloniert ist, wodurch man die Protein-kodierende DNA-Sequenz des NPT-II-Gens unter die Kontrolle der 5'- und 3'-Expressionssignale des Cauliflower-Mosaik-Gens VI stellt. Die so hergestellten Plasmide tragen die Bezeichnung pABDI und pABDII (vgl. Abbildung 1).

Beispiel 2: Transformation von Protoplasten von Nicotiana tabacum c.v. Petit Havanna SRI durch Uebertragung des NPT-II-Gens als Teil des Plasmids pABDI mit Hilfe der Mg$^{2+}$/PEG-Behandlung.

Tabakprotoplasten von Nicotiana tabacum c.v. Petit Havanna werden nach herkömmlichen Verfahren ausgehend von einer Tabak-Suspensionkultur hergestellt ([12] Potrykus I und Shillito RD, Methods in Enzymology, Vol 118, Plant Molecular Biology, eds. A. und H., Weissbach, Academic Press, Orlando, 1986). Vollständig entfaltete Blätter von 6 Wochen alten Spross-Kulturen werden unter sterilen Bedingungen entfernt und mit einer Enzym-Lösung der folgenden Zusammensetzung gründlich benetzt.

Enzymlösung: H$_2$O                                          70 ml

Sucrose                                          13 g

Macerozyme R 10                                  1 g

Cellulase                                        2 g

'Onozuka' R 10 (Yakult Co.

Ltd., Japan)

Drisellase (Chemische Fabrik

Schweizerhalle, Schweiz)                         0.13 g

2(n-Morpholin)-ethansulfonsäure

(MES)                                            0.5 ml

pH 6.0

Man zerschneidet die Blätter anschliessend in 1 - 2 cm grosse Quadrate und lässt sie auf der oben genannten Enzymlösung aufschwimmen. Die Inkubation erfolgt über Nacht bei einer Temperatur von 26°C im Dunkeln. Dieser Ansatz wird anschliessend leicht geschwenkt und für weitere 30 min bis zur vollständigen Verdauung inkubiert.

Diese Suspension wird dann durch ein Stahlsieb mit einer Maschenweite von 100 $\mu$m filtriert, mit 0.6 M Sucrose (MES, pH 5.6) durchgespült und anschliessend 10 Minuten bei 4000 - 5000 rpm zentrifugiert. Die Protoplasten sammeln sich auf der Oberfläche des Mediums, das dann unter den Protoplasten abgezogen wird, z.B. unter Verwendung einer sterilisierten Injektionsspritze.

Die Protoplasten werden in einem K3A Medium [Sucrose (102,96 g/l); Xylose (0.25 g/l); 2.4 Dichlorphenoxyessigsäure D (0.10 mg/l); 1-Naphtylessigsäure (1.00 mg/l); 6-Benzylaminopurin (0.20 mg/l); pH 5.8] [12) Potrykus I and Shillito, R.D. et al, 1981] resuspendiert, das 0.4 M Sucrose enthält.

Zur Durchführung der Transformationsexperimente werden die Protoplasten zunächst gewaschen, gezählt und anschliessend in einem W$_5$-Medium [154 mM NaCl, 125 mM CaCl$_2$ 2H$_2$O 5mM KCl, 5 mM Glucose, pH 5.6; 46) Menczel, L. et al. (1981)], das eine hohe Ueberlebensrate der isolierten Protoplasten gewährleistet, in einer Zelldichte von 1-2.5 x 10$^6$ Zellen pro ml resuspendiert. Die Protoplasten werden anschliessend nach einer Inkubationszeit von 30 Minuten bei 6 bis 8°C für die Transformationsexperimente verwendet.

Kurz vor der eigentlichen Transformation der isolierten Protoplasten wird das W$_5$-Medium durch das eigentliche Transformationsmedium ersetzt. Es handelt sich dabei um eine Mannit/Magnesium-Lösung [MaMg-Lsg: 0.4-0.5 mM Mannit, 0.1 % MES (Morpholinethansulfonsäure), pH 5.6] mit einer Mg$^{2+}$-Konzentration von 12 bis 16 mM. Dazu werden die Protoplasten zunächst aus der W$_5$-Lösung durch 5-minütige Zentrifugation bei 100 g abgetrennt und in dem MaMg-Medium resuspendiert (0.3 ml). Zu dieser Suspension werden dann 65 $\mu$l einer wässrigen DNA-Lösung, enthaltend 5-10 $\mu$g des Plasmids pABDI und 50 $\mu$g Kalbsthymus-Carrier DNA, zugesetzt. Bei letzterer handelt es sich um eine neutrale Carrier-DNA ohne transformierendes Insert, die zum Schutz vor einer Nukleaseverdauung der zu transformierende DNA zu diesem Ansatz im Ueberschuss hinzugegeben wird. Nach einer Zeitspanne von ca. 0.1 bis 10 Minuten nach der DNA-Zugabe erfolgt die Applikation einer wässrigen 40 %igen Polyethylenglykollösung (w/v) bis eine Endkonzentration von 24 bis 28 % (w/v) erreicht ist. Als besonders vorteilhaft erweist sich die Verwendung von PEG vom CMS Typ. Dabei handelt es sich um eine Ca$^{2+}$-haltige [0.1 M Ca(NO$_3$)$_2$•4H$_2$O] 0.4 M Mannitlösung, die PEG vom Molekulargewicht ca. 1000 bis ca. 6000 in einer Endkonzentration von 40 % (w/v) enthält. Der pH-Wert dieser Lösung liegt bei pH 7 bis 9 [15) Negrutiu, I. et al. (1986a)]

Im Falle von Nicotiana tabacum c.v. Petit Havanna SRI verwendet man vorzugsweise PEG CMS 4000. Der pH-Wert des Transformationsmediums wird anschliessend auf einen Wert von pH 7 eingestellt. Diese Mischung wird unter gelegentlichem Umschwenken für 30 Minuten bei 26°C inkubiert.

Bei Verwendung von hohen PEG-Konzentrationen von >20 % ist eine stufenweise Verdünnung mit dem 3 bis 5-fachen Volumen einer 0.2 M CaCl$_2$-Lösung vorteilhaft. Anschliessend werden die in der angegebenen Weise behandelten Protoplasten abzentrifugiert (5 Minuten bei 100 g) und in frischem K$_3$-Medium resuspendiert (0.3 ml Protoplastenlösung in 10 ml frisches K$_3$-Medium). Die weitere Inkubation erfolgt in 10 ml-Portionen in Petri-Schalen von 10 cm Durchmesser bei 24°C und Dunkelheit, wobei die Populationsdichte 4 bis 8x10$^4$ Protoplasten pro ml beträgt. Nach 3 Tagen wird das Kulturmedium pro Schale mit 0.3

Volumenteilen frischem $K_3$-Medium verdünnt und für weitere 4 Tage bei 24°C und 3000 lux künstlicher Beleuchtung inkubiert. Nach insgesamt 7 Tagen werden die aus den Protoplasten entwickelten Klone in mit 1 % Agarose verfestigtem, 50 mg/l Kanamycin enthaltendem Nährmedium eingebettet und nach der "bead type"-Kulturmethode [51) Shillito, R.D. et al., Plant Cell Reports, 2, 244-247 (1983)] bei 24°C unter Dunkelheit kultiviert. Das Nährmedium wird alle 5 Tage durch frische gleichartige Nährlösung ersetzt.

Beispiel 3: Regeneration Kanamycin-resistenter N. tabacum c.v. Petit Havana SR1

Nach 3 bis 4 Wochen fortgesetzter Kultivierung in Kanamycin-haltigem Nährmedium werden die resistenten Kalli von 2 bis 3 mm Durchmesser auf mit Agar verfestigtes LS-Kulturmedium [43) Linsmaier, E.M. and Skook, F., Physiol. Plant 18, 100-127 (1965)], enthaltend 0.05 mg/l 2,4-Dichlorphenoxyessigsäure, 2 mg/l 1-Naphthylessigsäure, 0.1 mg/l 6-Benzylaminopurin, 0.1 mg/l Kinetin und 75 mg/l Kanamycin, verpflanzt. Durch Sprossinduktion auf LS-Medium, enthaltend 150 mg/l Kanamycin und 0.2 mg/l 6-Benzylaminopurin, und anschliessender Wurzelbildung auf T-Medium [45) Nitsch, Y.P. and C. Nitsch, Science 163, 85-87 (1969)] erhält man Kanamycin-resistente Nicotiana tabacum-Pflanzen der Sorte Petit Havanna SRI.

Beispiel 4: Transformation von Protoplasten von Nicotiana plumbaginifolia durch Uebertragung des NPT-II-Gens als Teil des Plasmids pABDI mit Hilfe der $Mg^{2+}$/PEG-Behandlung.

Die Isolierung der Protoplasten sowie die Vorinkubation der für die eigentlichen Transformationsexperimente vorgesehenen Protoplasten erfolgt in genau analoger Weise zu dem für Nicotiana tabacum SR1 beschriebenen Verfahren.

Die eigentliche Transformation der in der angegebenen Weise vorbehandelten N. plumbaginifolia - Protoplasten wird in einer Mannit/Magnesium Lösung [MaMg-Lsg: 0.4 bis 0.5 mM Mannit, 0.1 % MES (Morpholinethansulfonsäure), pH 5.6] mit einer $Mg^{2+}$-Ionen-Konzentration von 22 bis 27 mM durchgeführt. Nach der Zugabe einer wässrigen DNA-Lösung (65 $\mu$l), enthaltend 5 bis 10 $\mu$g des Plasmids pABDI sowie 50 $\mu$g Kalbsthymus-Carrier-DNA, erfolgt die Applikation einer wässrigen 40 %igen Polyethylenglykollösung (w/v), bis eine Endkonzentration von 18 bis 22 % (w/v) erreicht ist.

Die Zeitspanne zwischen DNA-Zugabe und PEG Applikation beträgt in der Regel nur etwa 0.1 bis 10 Minuten. Auch im Falle von Nicotiana plumbaginifolia erweist sich die Verwendung von PEG CMS 4000 als besonders vorteilhaft für die Erzielung hoher Transformationsraten. Der pH-Wert des Transformationsmedium wird anschliessend auf einen Wert von pH 7 eingestellt. Es folgt eine ca. 30 minütige Inkubation dieser Transformationslösung bei einer Temperatur von 26°C unter gelegentlichem Umschwenken des Gemisches.

Die isolierten Protoplasten werden zunächst für einen Zeitraum von 4 bis 12 Tagen in einem Medium mit hoher Hormonkonzentration [($KA_1 AO$ - Medium, [42) Installe, P. et al., J. Plant, Physiol, 119, 443 - 454, (1985)] in einer Zelldichte von 2 bis 3 x $10^4$ Protoplasten/ml kultiviert.

Nach der Ausbildung von Zellaggregaten werden die überlebenden Kolonien ausgelesen, gezählt und in starker Verdünnung (0.5 bis 2 x $10^3$ Kolonien/ml in einem MDs-Medium [44) Negrutiu, I. et al., Theor. Appl. Genet. 66, 341 - 347, (1983)] oder einem $MAP_1 AO$ - Medium [42) Installe, P. et al., (1985)] mit niedrigem Hormongehalt, enthaltend 20 bis 40 mg/l Kanamycin-Sulphat, suspendiert. Anschliessend werden die Kolonien in Agar (1 % Agarose) eingebettet und nach der 'bead-type' [51) Shillito, R.D. et al., (1983)] Kultivierungsmethode bei einer Temperatur von 24°C und in Dunkelheit kultiviert. Das Nährmedium wird dabei alle 5 Tage durch frische gleichartige Kulturlösung ersetzt.

Beispiel 5: Regeneration Kanamycin-resistenter N. plumbaginfolia-Pflanzen

Nach einer Kultivierungsdauer von 3 bis 4 Wochen werden die resistenten Kalli, die einen Durchmesser von 2 - 5 mm erreicht haben, ausgelesen und auf festen Medien, enthaltend 50 mg/l Kanamycin, weitere 3 bis 5 Wochen kultiviert.

Die Regeneration ganzer Pflanzen erfolgt dabei entsprechend den Angaben bei [42) Installe, P. et al. (1985) durch Transfer der resistenten Kalli von einem RP-Medium auf ein R'SA und/oder ein M-Medium.

Beispiel 6: Nachweise des NPT-II-Gens im Pflanzenerbgut

0.5 g Kallus der transformierten Zellkulturen oder Blattgewebe der daraus regenerierten Pflanzen werden bei 0°C in 15 %-iger Saccharoselösung, enthaltend 50 mMol/l Ethylendiamin-N,N,N,N'-tetraessig-

14

säure, EDTA), 0.25 Mol/l Natriumchlorid und 50 mMol/l $\alpha,\alpha,\alpha$-Tris-(hydroxymethyl)-methylamin-Hydrochlorid (TRIS-HCl) bei pH 8.0 homogenisiert. Durch Zentrifugieren des Homogenisats für 5 Minuten bei 1000 g trennt man grob die Zellkerne ab. Die Zellkerne werden in 15 %-iger Saccharoselösung, enthaltend 50 mMol- EDTA und 50 mMol/l TRIS-HCl, bei pH 8.0 resuspendiert, mit Natrium-dodecylsulfat bis zu einer Endkonzentration von 0.2 % versetzt und für 10 Minuten auf 70°C erhitzt. Nach Abkühlung auf 20 bis 25°C wird der Mischung Kaliumacetat bis zu einer Konzentration von 0.5 Mol/l zugesetzt. Dieses Gemisch wird für 1 Stunde bei 0°C inkubiert. Der ausgefallene Niederschlag wird durch Zentrifugieren (15 Minuten bei 4°C in einer Mikrozentrifuge) abgetrennt. Aus der überstehenden Flüssigkeit wird durch Versetzen mit dem 2.5-fachen Volumen Aethanol bei 20 bis 25°C die DNA ausgefällt. Die abgetrennte DNA wird in einer Lösung von 10 mMol TRIS-HCl, enthaltend 10 $\mu$g/ml Ribonuclease A, gelöst, für 10 Minuten bei 37°C inkubiert, mit Proteinase K bis zu einer Konzentration von 250 $\mu$g/ml versetzt und für eine weitere Stunde bei 37°C inkubiert. Die Proteinase K wird durch Phenol- und Chloroform/Isoamylalkohol-Extraktionen enfernt. Aus der wässrigen Phase wird die NDA durch Zusatz von 0.6 Volumenteilen 0.6 molarer isopropanolischer Natriumacetat-Lösung ausgefällt und in 50 $\mu$l einer Lösung, enthaltend 10 mMol/l TRIS-HCl und 5 mMol/l EDTA, bei pH 7.5 gelöst. Durch diese Präparation erhält man DNA-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstücken des NPT-II-Gens und Vergleich mit dem Plasmid pABDI zeigt in einer "Southern Blot"-Analyse die Anwesenheit des NPT-II-Gens in der Zellkern-DNA der transformierten Nicotiana tabacum- sowie der Nicotiana plumbaginifolia-Zellen.

Ergebnisteil

Im folgenden werden die erzielten Transformationsergebnisse in Zusammenhang mit verschiedenen Transformationsparametern diskutiert.

Dabei werden die erreichten Transformationsraten in Form von 'Relativen Transformationsfrequenzen' (RTF) bzw. von 'Absoluten Transformationsfrequenzen' (ATF) wiedergegeben.

Bei den 'Relativen Transformationsfrequenzen' handelt es sich um das Verhältnis zwischen der Anzahl der Transformaten und der überlebenden Fraktion (die in der Lage ist Kolonien auszubilden) einer unselektiert kultivierten Protoplastenpopulation. Die 'Absolute Transformationsfrequenz' dagegen ist definiert als das Verhältnis zwischen derselben Anzahl an Transformanten und der ursprünglichen Zahl von Protoplasten, die vor der Transformation als lebend charakterisiert worden waren.

Ein Vergleich zwischen den ATF und RTF Werten vermittelt ein gutes Bild der Ueberlebensrate der Protoplasten nach Durchführung der einzelnen Transformations- und Selektionierungsschritte.

Einfluss von Struktur und Konzentration der verwendeten DNA auf die Transfomrationsrate

Tabelle 1: Einfluss der DNA-Struktur sowie des Konzentrationsverhältnisses zwischen Plasmid DNA (pABDI) und Carrier-DNA auf die Transformationsraten (RTF und ATF) bei Nicotiana plumbaginifolia Protoplasten.

Die Transformationsexperimente wurden in eine MaCa-Lösung bei einer PEG CMS4-Konzentration von 13 % durchgeführt.

| DNA-Konzentration [$\mu$g/ml] | GPPL x$10^6$ | UE x$10^4$ | ZT | RTF x$10^{-4}$ | ATF x$10^{-4}$ |
|---|---|---|---|---|---|
| Lineares pABDI | | | | | |
| 10 + 20 | 2 | 45 | 4 | 0.085 | 0.02 |
| 10 + 50 | 2 | 49.6 | 23 | 0.464 | 0.12 |
| 50 + 10 | 2 | 46.4 | 1 | 0.022 | 0.005 |
| Zirkuläres pABDI | | | | | |
| 10 + 50 | 2 | 45.4 | 0 | <0.01 | <0.005 |
| 50 + 10 | 2 | 41.6 | 0 | <0.01 | <0.005 |

Der Einfluss der DNA Struktur auf die Transformationsraten lässt sich am Beispiel von Nicotiana plumbaginifolia demonstrieren (Tab.1).

Es zeigt sich im Verlauf der durchgeführten Transformationsexperimente, dass sich bei Verwendung von linearer DNA deutlich höhere Transformationsfrequenzen erzielen lassen im Vergleich zu einer zirkulären DNA. Die erreichten Transformationsraten liegen dabei im Fall der linearen DNA um bis zu zwei Zehnerpotenzen höher als die entsprechenden Werte bei Verwendung einer zirkulären DNA.

Tabelle 1 macht deutlich, dass nicht nur die DNA Struktur sondern auch das Verhältnis zwischen Plasmid DNA und zugesetzter Carrier-DNA eine Rolle für die erreichbaren Transformationsfrequenzen spielen.

Die besten Resultate erreicht man, wenn die Carrier DNA deutlich im Ueberschuss vorliegt im Verhältnis zur Plasmid DNA, wobei ein Verhältnis von 10:50 (Plasmid DNA:Carrier DNA) besonders vorteilhaft ist.

Mit den hier erzielten Resultaten werden Ergebnisse bestätigt, die zuvor bereits für das Tabak-System ermittelt werden konnten [10) Shillito, R.D. et al., Bio/Technologie 3, (1985)].

2. Einfluss der PEG-Konzentration auf die Transformationsrate

Tabelle 2: Einfluss der PEG-Konzentration auf die Transformationsraten (RTF und ATF) von Nicotiana plumbaginifolia und N. tabacum c.v. Petit Havanna SR1 (C.S.) Protoplasten. Die Transformationsexperimente wurden in einer $W_5$-Salzlösung bei einem DNA-Konzentrationsverhältnis zwischen Plasmid- und Carrier-DNA von 1:5 durchgeführt.

| PEG-Konzentration [%, w/v] | GPPL | S | ZT | RTF x $10^{-4}$ | ATF x $10^{-4}$ |
|---|---|---|---|---|---|
| PEG CMS4 | | N. plumbaginifolia | | | |
| 8 % | 0.72 | - | 2 | 0.13 | 0.03 (±0.02) |
| 13 % | 1.05 | - | 7 | 0.26 | 0.063 (±0.035) |
| 20 % | 0.90 | - | 22 | 1.45 | 0.25 (±0.06) |
| 24 % | 1.0 | - | 32 | 2.0 | 0.32 (±0.07) |
| 27 % | 1.0 | - | 46 | 3.1 | 0.46 (±0.05) |
| PEG CMS6 | | N. tabacum, $SR_1$ | | | |
| 13 % | 0.33 | - | 14 | - | 0.28 (±0.15) |
| 20 % | 0.33 | - | 30 | - | 0.90 (±0.05) |
| 26 % | 0.33 | - | 40 | - | 1.2 (±0.08) |

Tabelle 2 verdeutlicht den Einfluss unterschiedlicher PEG Konzentrationen auf die erreichbaren Transformationsfrequenzen bei N. tabacum SR1 und N. plumbaginifolia. In beiden Fällen lässt sich ein eindeutiger Zusammenhang zwischen beiden Parametern feststellen. Mit der Zunahme der PEG-Konzentration von 8 % auf 27 % im Falle von N. plumbaginifola bzw. von 13 % auf 26 % bei N. tabacum SR1 geht ein Anstieg bei den erzielten Transformationsfrequenzen einher, die von 0.03 x $10^{-4}$ auf 0.46 x $10^{-4}$, bzw. von 0.28 x $10^{-4}$ auf 1.2 x $10^{-4}$ ansteigen.

3. Einfluss der $MgCl_2$-Konzentration auf die Transformationsrate

Tabelle 3: Einfluss der $MgCl_2$-Konzentration, des Applikationszeitpunktes, der verwendeten PEG-Lösung sowie, bei hohen PEG-Konzentrationen (> 25 %), der Waschbedingungen auf die Transformationsrate von N. tabacum SR1 Protoplasten. Für die Transformationsexperimente werden sowohl PEG CMS4 als auch PEG CMS6 Lösungen mit einer Endkonzentration von 20 % verwendet (Ausnahmen sind in der Tabelle gesondert angegeben).

| Transformation | GPPL x$10^6$ | ZT | ATF x$10^{-3}$ |
|---|---|---|---|
| **1. Kontrollen** | | | |
| W$_5$, kein Mg$^{2+}$ | 0.59 | 77 | 0.13 ± 0.03 |
| He Na/F, kein Mg$^{2+}$ | 0.26 | 265 | 1.0 ± 0.38 |
| MaMg 12mM, 8% PEG6R, | | | |
| 1500V, 3 Pulse, 1 KΩ, 10 nF | 0.26 | 324 | 1.3 ± 0.26 |
| MaMg 6mM, kein PEG | 0.33 | 0 | |
| Wie oben, + 0.1M MgCl$_2$ | | | |
| nach Zugabe von DNA | 0.33 | 0 | |
| **2. MgCl$_2$-Zugabe unmittelbar vor Transformation** | | | |
| W$_5$ + MaMg(1:1, MgCl$_2$ 4mM) | 0.37 | 1033 | 2.7 ± 0.46 |
| W$_5$ + MaMg(1:1, MgCl$_2$ 8mM) | 0.33 | 1087 | 3.3 ± 0.48 |
| MaMg 6mM | 0.50 | 2045 | 4.1 ± 0.51 |
| MaMg 12mM | 0.50 | 2056 | 4.1 ± 0.86 |
| **3. MgCl$_2$-Zugabe 2h vor Transformation** | | | |
| MaMg 6mM | 0.33 | 382 | 1.2 ± 0.30 |
| **4. PEG-Zusammensetzung (MaMg 12mM)** | | | |
| **PEG CMS 4(24%)** | 0.32 | 693 | 2.2 ± 0.55 |
| **PEG R6 (24%)** | 0.32 | 474 | 1.5 ± 0.34 |
| **5. Waschbedingungen (MaMg 12mM; 25% PEG)** | | | |
| CaCl$_2$.2H$_2$O 0.2M | 0.32 | 1127 | 3.5 ± 0.56 |
| W$_5$ | 0.32 | 686 | 2.1 ± 0.43 |

*MaMg.... mM gibt die MgCl$_2$-Konzentration in einer MaMg-Lösung an.

a) Applikationszeitpunkt: Wie aus Tabelle 3 ersichtlich, lassen sich die besten Ergebnisse mit einer Transformationsrate von 4,1 x $10^{-3}$ erzielen, wenn die Zugabe von MgCl$_2$ in Transformationsmedium unmittelbar vor der eigentlichen Transformation erfolgt.

Wird die Transformation dagegen erst 2 Stunden nach der erfolgten MgCl$_2$ Applikation durchgeführt, liegen die Transformationsraten nur noch bei Werten von 1.2 x $10^{-3}$.

Auch eine Kombination einer Mannit/MgCl$_2$-Lösung (MaMg-Lösung) mit einer W$_5$-Salzlösung in einem Mischungsverhältnis von 1:1 führt zu einer mehr oder weniger starken Abnahme der Transformationsfrequenz in Abhängigkeit von der molaren MgCl$_2$-Konzentration. Halbiert man beispielsweise die MgCl$_2$-Konzentration der MaMg-Lösung, so führt dies zu einem Rückgang der Transformationsrate um annähernd 20%.

Vergleicht man dagegen die Ergebnisse der Kontrollexperimente, die nur in Anwesenheit geeigneter Pufferlösungen (W$_5$- oder HeNa/F - Lösung) [39) Fromm, M. et al, Proc. Natl. Acad. Sci. USA 82, 5842 - 5848, (1985)] durchgeführt werden, aber in Abwesenheit von $Mg^{2+}$ Ionen, so erkennt man, dass in diesen Fällen die Transformationsfrequenzen um mehr als eine Zehnerpotenz niedriger liegen. Selbst bei Anwendung der Elektroparation lassen sich die zuvor diskutierten hohen Transformationsraten nicht erreichen.

PEG/$Mg^{2+}$ Synergismus: Die synergistische Wirkung bei gleichzeitiger Applikation von $Mg^{2+}$-Ionen und PEG zeigt sich sehr deutlich bei einem Vergleich der Transformationsergebnisse der Kontrollversuche, die in Anwesenheit von MgCl$_2$ (6mM) aber ohne Zusatz von PEG durchgeführt werden, sowie der eigentlichen Ausführungsbeispiele, bei denen MgCl$_2$ und PEG gemeinsam zur Anwendung kommen (Tab. 3).

Die Kontrollexperimente in Gegenwart von MgCl$_2$ in einer Konzentration von 6mM aber ohne Zusatz von PEG führen in keinem Fall zu nachweisbaren Transformationsereignissen.

Dagegen lassen sich bei Applikation von PEG (bei einer Endkonzentration von 20 %, w/v) unmittelbar nach (0.1 bis 10 min) der DNA-Zugabe in das MgCl enthaltende Transformationsmedium die oben bereits erwähnten hohen Transformationsraten von 4.1 x 10$^{-3}$ erreichen.

Diese Transformationsraten liegen damit auch um mehr als eine Zehnerpotenz höher als die vegleichbaren Resultate bei alleiniger Anwendung von PEG ohne MgCl$_2$ Zusatz (vgl. Tab. 2).

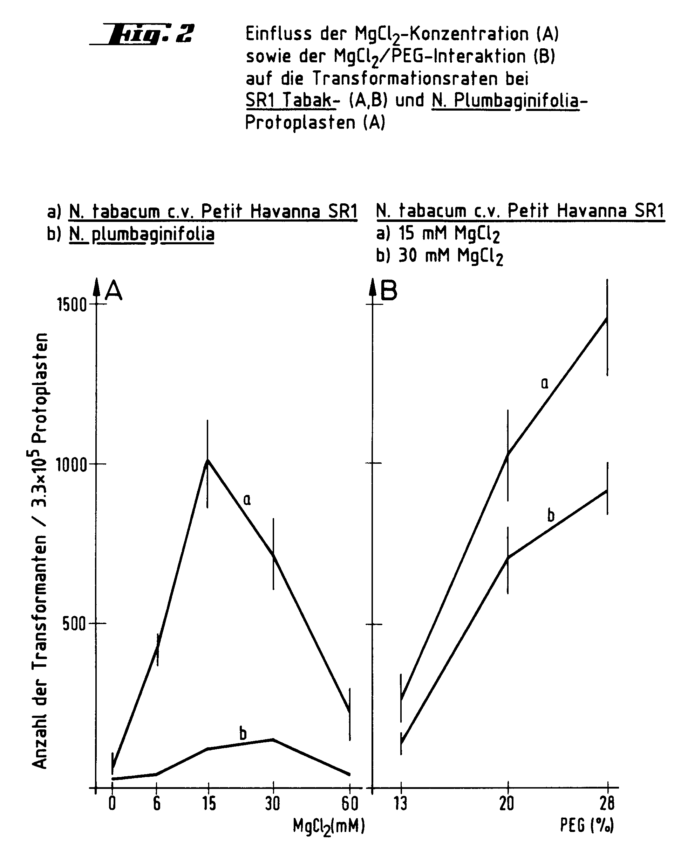

Im Gegensatz zum Tabak, bei dem maximale Transformationsraten in einem Konzentrationsbereich von 12 bis 15 mM MgCl$_2$ bei einer gleichzeitigen PEG CMS-Konzentration von 28 % erreicht werden (Abb. 2B), ist der Optimumsbereich bei N. plumbaginifolia zu höheren MgCl$_2$-Werten hin verschoben (s. Abb. 2A).

Maximale Transformationsraten werden in diesem Fall bei MgCl$_2$-Konzentrationen in einem Bereich zwischen 20 bis 30 mM und einer PEG-Konzentration von 20 % erreicht. Es lassen sich dabei Transformationsraten von 3.9 x 10$^{-4}$ erreichen.

PEG-Zusammensetzung: Neben den bereits erwähnten Parametern hat auch die Zusammensetzung der verwendeten PEG-Lösung einen Einfluss auf die erreichbaren Transforamtionsfrequenzen. Bei Anwendung von PEG CMS [15] Negrutiu, I. et al., (1986a) und PEG 6R [10] Shillito, R.D. et al., (1985) unter ansonsten gleichen Versuchsbedingungen (PEG - Konzentration 24 %, MgCl$_2$-Konzentration 12 mM) lassen sich im ersteren Fall deutlich höhere Transformationsraten erzielen, die um den Faktor 1.4 höher liegen.

Bei Anwendung hoher PEG-Konzentrationen von 24 % (20 % im Falle von N. plumbaginifolia) erweist es sich als vorteilhaft, die behandelten Protoplasten mit einer 0.2 M CaCl$_2$ x 2H$_2$O haltigen Lösung nachzuwaschen.

Durch diese Massnahme kann die Transformationsrate im Vergleich zu einer Behandlung mit eienr einfachen Salzlösung (W$_5$-Lösung) um den Faktor 1.7 gesteigert werden.

## 4. Nachweis der Uebertragung des transformierten Gens auf die sexuellen Nachkommenschaften sowie seiner Vererbung als normales Pflanzengen

Umfangreiche genetische Kreuzungsanalysen und ausführliche molekularbiologische Studien (beispielsweise "Southern blot"-Analyse der DNA des Pflanzengenoms; Untersuchung der Enzymaktivität der Aminoglykosidphosphotransferase, d.h., dem Enzym für die Kanamycin-spezifische Phosphorylierung) mit den genetisch veränderten Pflanzen (erste Generation und Nachkommenschaften) haben folgende Ergebnisse erbracht:

1. Das bakterielle Gen ist stabil in das Pflanzengenom eingebaut;
2. Es wird in der Regel unverändert und regelmässig auf Kreuzungsnachkommenschaftenübertragen;
3. Sein Erbgang entspricht dem eines natürlichen, einfachen, dominanten Pflanzengens;
4. Die molekulare Analyse mittels DNA-Hybridisierung und Enzymtest bestätigt die Ergebnisse der genetischen Kreuzungsanalyse;
5. Die genetisch veränderten Pflanzen haben ihren normalen, natürlichen Phänotyp während der Behandlung beibehalten; es sind also keine unerwünschten Veränderungen festgestellt worden.

Diese Ergebnisse zeigen, dass mit dem erfindungsgemässen Verfahren des direkten Gentransfers in Protoplasten der beste Weg für die gezielte genetische Veränderung von pflanzlichem Material aufgefunden worden ist. Die genetische Veränderung ist stabil, und unerwünschte Aenderungen des Genotyps der Pflange treten nicht auf.

Literatur:

1. Rothstein, S.J. and W.S. Reznikoff, Cell, 23, 191 - 199, (1981)
2. Herrera-Estrella, L. et al., Nature, 303, 209 - 213, (1983)
3. Bevan, M. W. and R.B. Flavell, Nature, 304, 184 -187 (1983)
4. Chilton, M.-D., Scientific American, 248(6),, 50 - 59 (1983)
5. Herrera-Estrella, L. et al., EMBO J., 2(6), 987 - 995, (1983)
6. Murai, N. et al., Science, 222, 476 - 482 (1983)
7. Harsch, R.B. et al., Science, 223, 496-498 (1984)
8. Fraley, R.T. et al., Proc. Natl. Acad. Sci. USA, 80, 4803 - 4807 (1983)
9. Potrykus I. et al., Plant Molec. Biol. Rep., 3 117 - 128, (1985)
10. Shillito, R.D. et al., Bio/Technology, 3, 1099 - 1103 (1985)
11. Neumann, E. et al., EMBO J., 7 841 - 845 (1982)
12. Potrykus I. and Shillito, R.D., Methods in Enzymology, 118, 449 - 578 (1986), Plant Molecular Biology, eds. A & H. Weissbach, Academic Press, Orlando (1986)
13. Koblitz, H., Methodische Aspekte der Zell- und Gewegezüchtung bei Gramineen unter besonderer Berücksichtigung der Getreide Kulturpflanze XXII, 93 - 157 (1974)

14. McCutcheon's Detergents and Emulsifiers Annual, Mc Publishing Corp., Ridgewood New Jersey, 1981; Stache, H., "Tensid-Taschenbuch", Carl Hanser Verlag, München/Wien, 1981
15. Negrutiu, I. et al., Theor. Appl. Genet., 72. 279 - 86 (1986a)
16. Wienand, U. et al., Mol. Gen. Genet, 182, 440 - 444 (1981)
17. Pennica, D. et al., Nature, 301, 214 - 221, (1983)
18. Stepien, P. et al., Gene, 24, 289 - 297, (1983)
19. Maniatis, T. et al., Molecular Cloning, Cold Spring Harbor Laboratory, 1982
20. Franck G. et al., Cell, 21: 285-294, 1980
21. Hohn et al., in: "Molecular Biology of Plant Tumors", Academic Press, New York, Seite 549-560, 1982
22. Berry-Lowe et al, J. Mol. Appl. Genet, 1: 483-498, 1982
23. Genetic Engineering of Plants, an Agricultural Perspective; ed. A. Cashmore, Plenum, New York 1983, Seite 29-38
24. Coruzzi G et al.; The Journal of Biological Chemistry, 258: 1399, 1983
25. Dunsmuir P et al., J. Mol. Appl. Genet., 2: 285, 1983
26. Gardner RC et al., Nucl. Acids Res, 9 (12): 2871-2888, 1981
27. Rodriguez, R.L. and Tait, R.C., Recombinant DNA techniques, Addison-Wesley Publishing Corp., London, Amsterdam, Don Mills, Ontario, Sydney, Tokyo, 1983
28. Messing, J. and Vieira, J., Gene, 19, 269 - 276, (1982)
29. Evans et al., "Protoplast Isolation and Culture", in Handbook of Plant Cell Culture, 1: 124-176 (Mac Millan Publishing Co., New York, 1983):
30. Davey MR, "Recent Developments in the Culture and Regeneration of Plant Protoplasts", Protoplasts, 1983 - Lecture Proceedings, Seite 19-29, (Birkhäuser, Basel, 1983)
31. Dale PJ, "Protoplast Culture and Plant Regeneration of Cereals and other Recalcitrant Crops", in Protoplasts 1983 - Lecture Proceedings, Seite 31-41 (Birkhäuser, Basel 1983)
32. Binding H, "Regeneration of Plants", in Plant Protolplasts, Seite 21-37, (CRC Press, Boca Raton, 1985).
33. Abdullah R el al., Bio/Technology, 4: 1087-1090, 1986
34. Fujimura T et al., Plant Tissue Culture Lett., 2: 74-75, 1985
35. Toriyama K. et al., Theor. Appl. Genet; 73: 16-19, 1986
36. Yamada Y. et al., Plant Cell Rep. 5: 85-88, 1986
37. Southern, E.M., J. Mol. Biol., 98, 503 - 517, (1975)
38. Rigby, W.J. et al., J. Mol. Biol., 113, 237 - 251, (1977)
39. Fromm, M. et al., Proc. Natl. Acad. Sci. USA, 82, 5842 - 5848, (1985)
40. Shillito, R.D. et al., Mutation Research, 81, 165 - 175, (1981)
41. Nagy, J.I and P. Maliga, Z. Pflanzenphysiologie, 78, 453 - 455 (1976)
42. Installe, P. et al., J. Plant Physiol., 119, 443 - 454, (1985)
43. Linsmakier, E.M. and Skook, F., Physiol. Plant, 18, 100 - 127, (1965)
44. Negrutiu, I. et al., Theor. Appl. Genet., 66, 341 - 347 (1983)
45. Nitsch, J.P. and Nitsch C., Science, 163, 85 - 87, (1969)
46. Menczel, L. et al., Theor. Appl. Genet, 59, 191 - 195, (1981)
47. Beck, E. et al., Gene, 19, 327 - 336, (1982)
48. Jacobsen, H. et al., Eur. J. Biochem., 45, 623, (1974)
49. Hohn B and Collins J., Gene, 11: 291-298, 1980
50. Soberon, X. et al., Gene, 9, 287 - 305, (1980)
51. Shillito, R.D. et al., Plant Cell Reports, 2, 244 - 247, (1983)

**Patentansprüche**

1. Verfahren zur Transformation pflanzlichen Erbgutes, dadurch gekennzeichnet, dass man
   - pflanzliche Protoplasten aus beliebigen Pflanzengeweben isoliert und in einem der üblicherweise für die Kultivierung pflanzlicher Protoplasten verwendeten Nährmedien kultiviert;
   - besagte Protoplasten vor der eigentlichen Transformation 20 Minuten bis 6 Stunden in einem Vorinkubationsmedium, enthaltend $Ca^{2+}$-, $K^+$- und $Na^+$- Ionen sowie eine geeignete C-Quelle, bei einer Temperatur von 4 bis 10°C vorinkubiert;
   - die Protoplasten anschliessend aus dem Vorinkubationsmedium abtrennt und in dem eigentlichen Transformationsmedium, enthaltend als essentiellen Bestandteil 0.1 bis 60 mM $Mg^{2+}$- Ionen in

19

An- oder Abwesenheit von $Ca^{2+}$- Ionen, resuspendiert;

- unmittelbar danach eine DNA-Probe, enthaltend ein oder mehrere Gene unter der Kontrolle von in Pflanzen aktiven Expressionssignalen sowie eine Träger-DNA, in die Transformationslösung hinein gibt;
- 0,1 bis 10 min. später ein die Plasmamembran modifizierendes Agenz hinzufügt und
- Protoplasten und DNA-Probe besagter Transformationslösung für einen Zeitraum inkubiert, der für die Aufnahme der DNA in die Protoplasten ausreicht.

2. Verfahren nach Anspruch 1, dadurch gekennzeichnet, dass besagte Transformationslösung $Mg^{2+}$-Ionen in einer Konzentration von 10 bis 30 mM enthält.

3. Verfahren nach Anspruch 1, dadurch gekennzeichnet, dass es sich bei dem die Plasmamembran modifizierenden Agenz um Polyethylenglykol handelt.

4. Verfahren nach Anspruch 3, dadurch gekennzeichnet, dass die Endkonzentration für Polyethylenglykol in besagtem Inkubationsmedium bei 10 % bis 30 % liegt.

5. Verfahren nach Anspruch 4, dadurch gekennzeichnet, dass die Endkonzentration für Polyethylenglykol bei 20 % bis 28 % liegt.

6. Verfahren nach Anspruch 1, dadurch gekennzeichnet, dass der pH-Wert der Inkubationslösung bei pH 5.6 bis pH 12 liegt.

7. Verfahren nach Anspruch 6, dadurch gekennzeichnet, dass der pH-Wert der Inkubationslösung bei pH 7 bis pH 10 liegt.

8. Verfahren nach Anspruch 1, dadurch gekennzeichnet, dass die Konzentration der DNA-Probe, enthaltend ein oder mehrere Gene beliebigen Ursprungs unter der Kontrolle von in Pflanzen aktiven Expressionssignalen sowie eine das Gen flankierende neutral, d.h. an der Funktion des Gens nicht beteiligte DNA-Sequenz, 2 bis 20 $\mu$g/ml beträgt.

9. Verfahren nach Anspruch 8, dadurch gekennzeichnet, dass die Konzentration besagter DNA- Probe 5 bis 10 $\mu$g/ml beträgt.

10. Verfahren nach Anspruch 1, dadurch gekennzeichnet, dass der Transformationslösung zusätzlich neutrale DNA ohne transformierendes Gen als Carrier DNA im Ueberschuss zugesetzt wird.

11. Verfahren nach Anspruch 10, dadurch gekennzeichnet, dass es sich bei besagter neutraler DNA um tierische DNA, pflanzliche DNA, λ-DNA, Plasmid DNA oder jede beliebige für die Durchführung des Verfahrens geeignete DNA handelt.

12. Verfahren nach einem der Ansprüche 10 oder 11, dadurch gekennzeichnet, dass es sich bei besagter neutraler DNA um Kalbsthymus-Carrier-DNA handelt.

13. Verfahren nach Anspruch 10, dadurch gekennzeichnet, dass die Konzentration der Carrier DNA 50 bis 70 $\mu$g/ml beträgt.

14. Verfahren nach Anspruch 1, dadurch gekennzeichnet, dass die Vorinkubation der Protoplasten über einen Zeitraum von 20 Minuten bis 1 Stunde erfolgt.

15. Verfahren nach Anspruch 1, dadurch gekennzeichnet, dass die gemeinsame Inkubation von Protoplasten und DNA in Gegenwart eines die Plasmamembran modifizierenden Agens im eigentlichen Transformationsmedium, enthaltend als essentielle Bestandteile 0.1 bis 60 mM $Mg^{2+}$-Ionen in An- oder Abwesenheit von $Ca^{2+}$-Ionen, über einen Zeitraum von 10 Minuten bis 6 Stunden erfolgt.

16. Verfahren gemäss Anspruch 1, dadurch gekennzeichnet, dass man als transformierendes Gen ein Gen verwendet, dessen Strukturteil pflanzlicher, tierischer, mikrobieller, viraler oder synthetischer Herkunft ist und dessen Expressionssignale pflanzlicher, tierischer oder synthetischer Herkunft sind.

**17.** Verfahren gemäss Anspruch 16, dadurch gekennzeichnet, dass besagtes transformierendes Gen entweder aus genomischer DNA aus einer cDNA oder aus synthetischer DNA besteht.

**18.** Verfahren gemäss Anspruch 16, dadurch gekennzeichnet, dass besagtes transformierendes Gen sowohl aus genomischer DNA, als auch aus cDNA und/oder synthetischer DNA zusammengesetzt ist

**19.** Verfahren gemäss Anspruch 16, dadurch gekennzeichnet, dass das transformierende Gen aus Genfragmenten von mehreren Organismen, die verschiedenen Gattungen angehören, zusammengesetzt ist.

**20.** Verfahren gemäss Anspruch 16, dadurch gekennzeichnet, dass das transformierende Gen aus Genfragmenten von mehr als einem Stamm, einer Varietät oder einer Spezies des gleichen Organismus zusammengesetzt ist.

**21.** Verfahren gemäss Anspruch 16, dadurch gekennzeichnet, dass das transformierende Gen aus Anteilen von mehr als einem Gen des selben Organismus zusammengesetzt ist.

**22.** Verfahren gemäss Anspruch 16, dadurch gekennzeichnet, dass besagtes transformierendes Gen einen Strukturteil besitzt, der der transformierenden Pflanze eine nützliche und wünschenswerte Eigenschaft verleiht.

**23.** Verfahren gemäss Anspruch 22, dadurch gekennzeichnet, dass besagtes Strukturgen der Pflanze eine erhöhte Resistenz oder Toleranz gegenüber Pathogenen, Herbiziden, Insektiziden und/oder anderen Bioziden verleiht.

**24.** Verfahren gemäss Anspruch 22, dadurch gekennzeichnet, dass besagtes Strukturgen die Bildung und Qualität von Reserve- und Lagerstoffen in Blättern, Samen, Knollen, Wurzeln und Stengeln verbessert.

**25.** Verfahren gemäss Anspruch 22, dadurch gekennzeichnet, dass besagtes Strukturgen für pharmazeutisch akzeptable aktive Wirksubstanzen kodiert.

**26.** Verfahren gemäss Anspruch 16, dadurch gekennzeichnet, dass besagt Expressionssignale aus Genen von Pflanzen oder Pflanzenviren stammen.

**27.** Verfahren gemäss Anspruch 16, dadurch gekennzeichnet, dass besagte Expressionssignale aus einem pflanzlichen Gen stammen, das für die kleine Untereinheit der Ribulosebisphosphatcarboxylase oder das Chlorphyle a/b Bindungsprotein kodiert.

**28.** Verfahren gemäss Anspruch 16, dadurch gekennzeichnet, dass besagt Expressionssignale aus Genen von Pflanzenviren stammen.

**29.** Verfahren gemäss Anspruch 28, dadurch gekennzeichnet, dass es sich um Cauliflower Mosaik Virus (CaMV) handelt.

**30.** Verfahren gemäss Anspruch 28, dadurch gekennzeichnet, dass es sich um die 35 S-Expressionssignale des CaMV Genoms handelt.

**31.** Verfahren gemäss Anspruch 29, dadurch gekennzeichnet, dass es sich um die 19 S-Expressionssignale des CaMV Genoms handelt.

**32.** Verfahren gemäss Anspruch 1, dadurch gekennzeichnet, dass als pflanzliche Protoplasten solche einer einzigen Pflanzenart oder einer der Art untergeordneten systematischen Einheit verwendet werden.

**33.** Verfharen gemäss Anspruch 32, dadurch gekennzeichnet, dass besagte Protoplasten aus Blättern, Keimlingen, Stengeln, Blüten, Wurzeln, Pollen oder Embryonen gewonnen werden.

**34.** Verfahren gemäss Anspruch 33, dadurch gekennzeichnet, dass es sich um Blattprotoplasten handelt.

EP 0 270 496 B1

**35.** Verfahren gemäss Anspruch 32, dadurch gekennzeichnet, dass besagte Protoplasten aus Zellkulturen gewonnen werden.

**36.** Verfahren gemäss Anspruch 1, dadurch gekennzeichnet, dass es sich um Protoplasten von Pflanzen der Familien Solanaceae, Crucifrea, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae oder Gramineae oder der Ordnung Leguminosae handelt.

**37.** Verfahren gemäss Anspruch 1, dadurch gekennzeichnet, dass es sich um Protoplasten der Familien Solanaceae, Cruciferae und Gramineae handelt.

**38.** Verfahren gemäss Anspruch 1, dadurch gekennzeichnet, dass es sich um Protoplasten von Mais, Reis, Weizen, Gerste, Roggen, Hafer oder Hirse handelt.

**39.** Verfahren gemäss Anspruch 1, dadurch gekennzeichnet, dass es sich um Protoplasten der Gattungen Solanum, Nicotiana, Brassica, Beta, Pisum, Phaseolus, Glycine, Helianthus, Allium, Avena, Hordeum, Oryzae, Setaria, Secale, Sorghum, Triticum, Zea, Musa, Cocos, Cydonia, Pyrus, Malus, Phoenix, Elaeis, Rubus, Fragaria, Prunus, Arachis, Secale, Panicum, Saccharum, Coffea, Camellia, Musa, Ananas, Vitis oder Citrus handelt.

**40.** Verfahren gemäss Anspruch 1 zur Transformation von Pflanzen, welche aus Protoplasten regenerierbar sind.

**41.** Verfahren gemäss Anspruch 1 zur Transformation von Pflanzen der Gruppe Nicotiana tabacum, Nicotiana plumbaginifolia, Petunia hybrida, Hyoscyamus muticus und Brassica napus mittels Uebertragung des NPT-II-Gens, dadurch gekennzeichnet, dass man das NPT-II-Gen mit Promoter- und Terminationssignalen des CaMV-Gens VI versieht, dieses in das pUC8-Plasmid einbaut und das erhaltene chimäre Plasmid durch $Mg^{2+}$/Polyethylenglykolbehandlung in isolierte Protoplasten von Pflanzen der vorstehend genannten Gruppe überträgt.

**Claims**

**1.** A process for the transformation of a plant genotype, wherein
- plant protoplasts are isolated from any plant tissue and cultured in one of the nutrient media customarily used for culturing plant protoplasts;
- before the actual transformation, the said protoplasts are pre-incubated at a temperature of from $4°$ to $10°C$ for from 20 minutes to 6 hours in a pre-incubation medium containing $Ca^{2+}$, $K^+$ and $Na^+$ ions and also a suitable carbon source;
- the protoplasts are then separated from the pre-incubation medium and resuspended in the actual transformation medium which contains as an essential component from 0.1 to 60 mM $Mg^{2+}$ ions in the presence or absence of $Ca^{2+}$ ions;
- directly thereafter a DNA sample, containing one or more genes under the control of expression signals active in plants and also a carrier DNA, is added to the transformation solution;
- from 0.1 to 10 minutes later, a plasma membrane-modifying agent is added, and
- the protoplasts and DNA sample are incubated in the said transformation solution for a period that is sufficient for the incorporation of the DNA into the protoplasts.

**2.** A process according to claim 1, wherein the said transformation solution contains $Mg^{2+}$ ions in a concentration of from 10 to 30 mM.

**3.** A process according to claim 1, wherein the plasma membrane-modifying agent is polyethylene glycol.

**4.** A process according to claim 3, wherein the final concentration of polyethylene glycol in the said incubation medium is from 10 % to 30 %.

**5.** A process according to claim 4, wherein the final concentration of polyethylene glycol is from 20 % to 28 %.

22

6. A process according to claim 1, wherein the pH value of the incubation solution is from pH 5.6 to pH 12.

7. A process according to claim 6, wherein the pH value of the incubation solution is from pH 7 to pH 10.

8. A process according to claim 1, wherein the concentration of the DNA sample, containing one or more genes of any origin under the control of expression signals that are active in plants and also containing a neutral DNA sequence flanking the gene, that is to say a DNA sequence that does not participate in the function of the gene, is from 2 to 20 $\mu$g/ml.

9. A process according to claim 8, wherein the concentration of the said DNA sample is from 5 to 10 $\mu$g/ml.

10. A process according to claim 1, wherein, in addition, neutral DNA without a transforming gene is added in excess as carrier DNA to the transformation solution.

11. A process according to claim 10, wherein the said neutral DNA is animal DNA, plant DNA, $\lambda$-DNA, plasmid DNA or any DNA suitable for carrying out the process.

12. A process according to either claim 10 or claim 11, wherein the said neutral DNA is calf thymus carrier DNA.

13. A process according to claim 10, wherein the concentration of the carrier DNA is from 50 to 70 $\mu$g/ml.

14. A process according to claim 1, wherein the pre-incubation of the protoplasts is carried out over a period of from 20 minutes to 1 hour.

15. A process according to claim 1, wherein the protoplasts and DNA are jointly incubated in the actual transformation medium, containing as essential component from 0.1 to 60 mM $Mg^{2+}$ ions in the presence or absence of $Ca^{2+}$ ions, in the presence of a plasma membrane-modifying agent, for a period of from 10 minutes to 6 hours.

16. A process according to claim 1, wherein the transforming gene used is a gene of which the structural part is of plant, animal, microbial, viral or synthetic origin and of which the expression signals are of plant, animal or synthetic origin.

17. A process according to claim 16, wherein the said transforming gene consists of genomic DNA, of a cDNA or of synthetic DNA.

18. A process according to claim 16, wherein the said transforming gene is composed both of genomic DNA, and of cDNA and/or synthetic DNA.

19. A process according to claim 16, wherein the transforming gene is composed of gene fragments from several organisms that belong to different genera.

20. A process according to claim 16, wherein the transforming gene is composed of gene fragments from more than one strain, one variety or one species of the same organism.

21. A process according to claim 16, wherein the transforming gene is composed of portions of more than one gene from the same organism.

22. A process according to claim 16, wherein the said transforming gene has a structural part that imparts a useful and desirable property to the plant being transformed.

23. A process according to claim 22, wherein the said structural gene imparts to the plant an increased resistance to, or tolerance of, pathogens, herbicides, insecticides and other biocides.

**24.** A process according to claim 22, wherein the said structural gene improves the formation and quality of reserve and stored substances in leaves, seeds, tubers, roots and stems.

**25.** A process according to claim 22, wherein the said structural gene codes for a pharmaceutically acceptable active substance.

**26.** A process according to claim 16, wherein the said expression signals originate from genes of plants or plant viruses.

**27.** A process according to claim 16, wherein the said expression signals originate from a plant gene that codes for the small subunit of ribulosediphosphate carboxylase or for the chlorophyll a/b binding protein.

**28.** A process according to claim 16, wherein the said expression signals originate from genes of plant viruses.

**29.** A process according to claim 28, wherein the plant virus is Cauliflower Mosaic Virus (CaMV).

**30.** A process according to claim 28, wherein the expression signals are 35S expression signals of the CaMV genome.

**31.** A process according to claim 29, wherein the expression signals are 19S expression signals of the CaMV genome.

**32.** A process according to claim 1, wherein the plant protoplasts used are those from a single species or from a systematic unit subordinate to the species.

**33.** A process according to claim 32, wherein the said protoplasts are obtained from leaves, seedlings, stems, flowers, roots, pollen or embryos.

**34.** A process according to claim 33, wherein the protoplasts are leaf protoplasts.

**35.** A process according to claim 32, wherein the said protoplasts are obtained from cell cultures.

**36.** A process according to claim 1, wherein the protoplasts are from plants of the Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae or Gramineae families or of the order Leguminosae.

**37.** A process according to claim 1, wherein the protoplasts are of the Solonaceae, Cruciferae and Gramineae families.

**38.** A process according to claim 1, wherein the protoplasts are of maize, rice, wheat, barley, rye, oats or millet.

**39.** A process according to claim 1, wherein the protoplasts are of the genera Solanum, Nicotiana, Brassica, Beta, Pisum, Phaseolus, Glycine, Helianthus, Allium, Avena, Hordeum, Oryzae, Setaria, Secale, Sorghum, Triticum, Zea, Musa, Cocos, Cydonia, Pyrus, Malus, Phoenix, Elaeis, Rubus, Fragaria, Prunus, Arachis, Secale, Panicum, Saccharum, Coffea, Camellia, Musa, Ananas, Vitis or Citrus.

**40.** A process according to claim 1 for the transformation of plants that can be regenerated from protoplasts.

**41.** A process according to claim 1 for the transformation of plants from the group Nicotiana tabacum, Nicotiana plumbaginifolia, Petunia hybrida, Hyoscyamus muticus and Brassica napus by means of transfer of the NPT-II gene, wherein the NPT-II gene is provided with promoter and termination signals of the CaMV gene VI, this is inserted into the pUC8 plasmid and the resulting chimaeric plasmid is

24

transferred by $Mg^{2+}$/polyethylene glycol treatment into isolated protoplasts of plants from the group mentioned above.

**Revendications**

1. Procédé pour la transformation de matériel héréditaire végétal, caractérisé en ce que
   - on isole des protoplastes végétaux à partir de tissus végétaux quelconques, et on les cultive dans un milieu nutritif habituellement utilisé pour la culture de protoplastes végétaux;
   - on met à préincuber lesdits protoplastes, avant la transformation proprement dite, à une température de 4 à 10°C pendant 20 minutes à 6 heures, dans un milieu de préincubation contenant des ions $Ca^{2+}$, $K^+$ et/ou $Na^+$, ainsi qu'une source de carbone appropriée;
   - on sépare ensuite les protoplastes du milieu de préincubation et on les remet en suspension dans le milieu de transformation proprement dit, contenant, en tant que composant essentiel, de 0,1 à 60 mM d'ions $Mg^{2+}$, en présence ou en absence d'ions $Ca^{2+}$;
   - immédiatement après cela, on introduit dans la solution de transformation un échantillon d'ADN contenant un ou plusieurs gènes sous le contrôle de signaux d'expression actifs dans des végétaux, ainsi qu'un ADN vecteur;
   - 0,1 à 10 minutes plus tard, on ajoute un agent modifiant la membrane plasmatique; et
   - on met à incuber les protoplastes et l'échantillon d'ADN, dans ladite solution de transformation, pendant une durée suffisante pour la fixation de l'ADN dans les protoplastes.

2. Procédé selon la revendication 1, caractérisé en ce que ladite solution de transformation contient des ions $Mg^{2+}$ à une concentration de 10 à 30 mM.

3. Procédé selon la revendication 1, caractérisé en ce que l'agent modificateur de la membrane plasmatique est le polyéthylèneglycol.

4. Procédé selon la revendication 3, caractérisé en ce que la concentration finale du polyéthylèneglycol dans ledit milieu d'incubation va de 10 à 30 %.

5. Procédé selon la revendication 4, caractérisé en ce que la concentration finale du polyéthylèneglycol va de 20 à 28 %.

6. Procédé selon la revendication 1, caractérisé en ce que le pH de la solution d'incubation est dans la plage de 5,6 à 12.

7. Procédé selon la revendication 6, caractérisé en ce que le pH de la solution d'incubation est dans la plage de 7 à 10.

8. Procédé selon la revendication 1, caractérisé en ce que la concentration de l'échantillon d'ADN, contenant un ou plusieurs gènes d'origine quelconque, sous le contrôle de signaux d'expression actifs dans des végétaux ainsi qu'une séquence d'ADN neutre, encadrant le gène, c'est-à-dire ne participant pas à la fonction du gène, va de 2 à 20 $\mu$g/ml.

9. Procédé selon la revendication 8, caractérisé en ce que la concentration dudit échantillon d'ADN va de 5 à 10 $\mu$g/ml.

10. Procédé selon la revendication 1, caractérisé en ce que, en outre, on ajoute en excès à la solution de transformation de l'ADN neutre, sans gène transformant, en tant qu'ADN vecteur.

11. Procédé selon la revendication 10, caractérisé en ce que, en ce qui concerne ledit ADN neutre, il s'agit d'ADN animal, d'ADN végétal, d'ADN de λ, d'ADN plasmidique ou de tout ADN approprié à la mise en oeuvre du procédé.

12. Procédé selon la revendication 10 ou 11, caractérisé en ce que, ledit ADN neutre est un ADN vecteur de thymus de veau.

13. Procédé selon la revendication 10, caractérisé en ce que la concentration de l'ADN vecteur va de 50 à 70 $\mu$g/ml.

14. Procédé selon la revendication 1, caractérisé en ce que la préincubation des protoplastes s'effectue pendant une durée de 20 minutes à 1 heure.

15. Procédé selon la revendication 1, caractérisé en ce que l'incubation simultanée des protoplastes et d'ADN est effectuée en présence d'un agent modifiant la membrane plasmatique, dans le milieu de transformation proprement dit, contenant, en tant que composants essentiels, de 0,1 à 60 mM d'ions $Mg^{2+}$, en présence ou en absence d'ions $Ca^{2+}$, pendant une durée de 10 minutes à 6 heures.

16. Procédé selon la revendication 1, caractérisé en ce que, en tant qu'agent transformant, on utilise un gène dont la partie structurale est d'origine végétale, animale, microbienne, virale ou synthétique, et dont les signaux d'expression sont d'origine végétale, animale ou synthétique.

17. Procédé selon la revendication 16 caractérisé en ce que ledit gène transformant consiste soit en ADN génomique, soit en un ADNc ou en ADN synthétique.

18. Procédé selon la revendication 16, caractérisé en ce que ledit gène transformant est composé aussi bien d'ADN génomique que d'ADNc et/ou d'ADN synthétique.

19. Procédé selon la revendication 16, caractérisé en ce que le gène transformant est composé de fragments géniques de plusieurs organismes qui appartiennent à des genres différents.

20. Procédé selon la revendication 16, caractérisé en ce que le gène transformant est composé de fragments de gène de plus d'une souche, d'une variété ou d'une espèce du même organisme.

21. Procédé selon la revendication 16, caractérisé en ce que le gène transformant est composé de fragments de plus d'un gène du même organisme.

22. Procédé selon la revendication 16, caractérisé en ce que ledit gène transformant possède une partie structurale qui confère aux végétaux transformés une propriété utile et souhaitable.

23. Procédé selon la revendication 22, caractérisé en ce que ledit gène structural confère aux végétaux une résistance ou tolérance accrue à l'égard de pathogènes, herbicides, insecticides et/ou autres biocides.

24. Procédé selon la revendication 22, caractérisé en ce que ledit gène structural améliore la formation et la qualité de substance de réserve ou de stockage dans des feuilles, graines, tubercules, racines et tiges.

25. Procédé selon la revendication 22, caractérisé en ce que ledit gène structural code pour des substances actives pharmaceutiquement acceptables.

26. Procédé selon la revendication 16, caractérisé en ce que lesdits signaux d'expression proviennent de gènes de végétaux ou de virus de végétaux.

27. Procédé selon la revendication 16, caractérisé en ce que lesdits signaux d'expression proviennent d'un gène végétal qui code pour une petite sous-unité de la ribulosebisphosphate carboxylase ou de la protéine de liaison des chlorophylles a/b.

28. Procédé selon la revendication 16, caractérisé en ce que lesdits signaux d'expression proviennent de gènes de virus de végétaux.

29. Procédé selon la revendication 28, caractérisé en ce qu'il s'agit du virus de la mosaïque du chou-fleur (CaMV).

30. Procédé selon la revendication 18, caractérisé en ce qu'il s'agit des signaux d'expression 35S du génome de CaMV.

**31.** Procédé selon la revendication 29, caractérisé en ce qu'il s'agit des signaux d'expression 19S du génome de CaMV.

**32.** Procédé selon la revendication 1, caractérisé en ce que, en tant que protoplastes végétaux, on utilise ceux d'une seule espèce végétale ou d'une unité systématique subordonnée de l'espèce.

**33.** Procédé selon la revendication 32, caractérisé en ce que lesdits protoplastes sont obtenus à partir de feuilles, plantules, tiges, fleurs, racines, pollen ou embryons.

**34.** Procédé selon la revendication 33, caractérisé en ce qu'il s'agit de protoplastes de feuilles.

**35.** Procédé selon la revendication 32, caractérisé en ce que lesdit protoplastes sont obtenus à partir de cultures de cellules.

**36.** Procédé selon la revendication 1, caractérisé en ce qu'il s'agit de protoplastes de plantes appartenant aux familles des solanées, crucifères, composées, liliacées, vitacées, chénopodiacées, rutacées, alliacées, amaryllidacées, asparagacées, orchidacées, palmacées, broméliacées, rubiacées, théacées, musacées ou graminées, ou à l'ordre des légumineuses.

**37.** Procédé selon la revendication 1, caractérisé en ce qu'il s'agit de protoplastes des familles des solanées, crucifères et graminées.

**38.** Procédé selon la revendication 1, caractérisé en ce qu'il s'agit de protoplastes de maïs, riz, blé, orge, seigle, avoine ou millet.

**39.** Procédé selon la revendication 1, caractérisé en ce qu'il s'agit de protoplastes appartenant aux genres *Solanum, Nicotiana, Brassica, Beta, Pisum, Phaseolus, Glycine, Helianthus, Allium, Avena, Hordeum, Oryzae, Setaria, Secale, Sorghum, Triticum, Zea, Musa, Cocos, Cydonia, Pyrus, Malins, Phoenix, Elaeis, Rubus, Fragaria, Prunus, Arachis, Panicum, Saccharum, Coffea, Camellia, Ananas, Vitis ou Citrus.*

**40.** Procédé selon la revendication 1, pour la transformation de végétaux qui peuvent être régénérés à partir de protoplastes.

**41.** Procédé selon la revendication 1, pour la transformation de végétaux appartenant aux groupes *Nicotiana tabacum, Nicotiana plumbaginifolia, Petunia hybrida, Hyoscyamus muticus* et *Brassica napus,* par transfert du gène NPT-II, caractérisé en ce que l'on munit le gène NPT-II de signaux promoteurs et de terminaison du gène VI de CaMV, on incorpore celui-ci dans le plasmide pUC8 et on transfère le plasmide chimère obtenu, par traitement au polyéthylèneglycol/Mg$^{2+}$, dans des protoplastes isolés de végétaux appartenant au groupe précité.

**Fig. 1** Herstellung der Plasmide pABD I und pABD II

///////// : NPT II -STRUKTURGEN

4630 4833 5383 5708  5848                    7340 7670
BglII SalI PstI EcoRV HindIII              EcoRV BglII

GEN V        GEN VI

TAA ATG                              TGA

## GEN VI  REGION des Cauliflower Mosaik Virus

HindIII PstI

pkm 21        TGA

PstI                    HindIII

ATG    pkm 244    TGA

HindIII PstI                HindIII

pkm 21244   TGA

EcoRV
PstI    HindIII                HindIII
BamHI                                    BamHI

ATG   pJPAX Cakm    TGA

PstI EcoRV                  HindIII   BamHI
BamHI        HindIII                    EcoRV

ATG  pBR 327 Cakm    TGA

EcoRV
SalI PstI EcoRV              HindIII   BglII
HindIII                                SalI

ATG   pABDI(5.3kb)   TGA

EcoRV                  EcoRV
SalI PstI  HindIII        HindIII  BglII
SalI

ATG   pABD II (5.3kb)

**Fig. 2**  Einfluss der MgCl$_2$-Konzentration (A)
sowie der MgCl$_2$/PEG-Interaktion (B)
auf die Transformationsraten bei
SR1 Tabak- (A,B) und N. Plumbaginifolia-
Protoplasten (A)

a) N. tabacum c.v. Petit Havanna SR1
b) N. plumbaginifolia

N. tabacum c.v. Petit Havanna SR1
a) 15 mM MgCl$_2$
b) 30 mM MgCl$_2$